# EUROPEAN PATENT SPECIFICATION

(11) **EP 3 565 585 B1**
(45) Date of publication and mention of the grant of the patent: **13.11.2024**
(21) Application number: 18701797.5
(22) Date of filing: 04.01.2018
(51) Int. Cl.: A61K 39/00, A61K 38/17, A61P 25/00

(54) **THERAPEUTIC METHOD USING DOSE ESCALATION PROTOCOL FOR TOLEROGENIC PEPTIDES**
THERAPEUTISCHE METHODE MIT DOSISESKALATIONSPROTOKOLL FÜR TOLEROGENE PEPTIDE
MÉTHODE THÉRAPEUTIQUE UTILISANT UN PROTOCOLE D'ESCALADE DE LA DOSE POUR LES PEPTIDES TOLÉROGÈNES

(30) Priority: 04.01.2017 GB 201700111
(43) Date of publication of application: 13.11.2019
(73) Proprietor: Worg Pharmaceuticals (Zhejiang) Co., Ltd., Huzhou, Zhejiang (CN)
(72) Inventor: WRAITH, David, Chepstow Gwent NP16 5UH (GB); MARTIN, Keith, Chepstow Gwent NP16 5UH (GB)
(74) Representative: D Young & Co LLP
(86) International application number: PCT/IB2018/050061
(87) International publication number: WO 2018/127828

(56) References cited:
- WO-A1-03/064464
- WO-A1-2016/103213
- WO-A2-02/16410
- WO-A2-2009/056833
- WO-A2-2015/019302
- AU-A1- 2006 203 165
- ES-T3- 2 370 957
- HEATHER B STREETER ET AL: "Preclinical development and first-in-human study of ATX-MS-1467 for immunotherapy of MS", NEUROLOGY: NEUROIMMUNOLOGY & NEUROINFLAMMATION, vol. 2, no. 3, 1 June 2015 (2015-06-01), US, pages e93 - e93, XP055461476, ISSN: 2332-7812, DOI: 10.1212/NXI.0000000000000093

## Description

### FIELD OF THE INVENTION

The present invention relates to a therapeutic method using a tolerogenic peptide. In particular, the invention relates to a dosage regimen for a tolerogenic peptide. The therapeutic method can be used to treat or prevent conditions associated with aberrant, hypersensitivity or pathological immune responses to endogenous or exogenous proteins that results in a loss of immune tolerance.

### BACKGROUND

The immune system is a system within an organism that protects against disease. To function properly, an immune system must detect a wide variety of pathogens, from viruses to parasitic worms, and distinguish them from the organism's own healthy tissue.

However, whilst an immunological response may be elicited to foreign antigens, it is not desirable that an immune response is elicited from the organism's own "self" tissue. The phenomenon of "immune tolerance" or "immunological tolerance" has evolved to prevent aberrant or pathological immune responses.

Immune tolerance describes a state of unresponsiveness of the immune system to substances or tissue that have the capacity to elicit an immune response. The terminology "immune tolerance" encompasses a range of physiological mechanisms by which the body reduces or eliminates an immune response to particular agents. It is used to describe the phenomenon underlying discrimination of self from non-self, suppressing allergic responses, allowing chronic infection instead of rejection and elimination, and preventing attack of foetuses by the maternal immune system.

Natural or "self" tolerance is the failure to attack the body's own proteins and other antigens. If the immune system should respond to "self", an autoimmune disease may result. Thus, immune tolerance can malfunction and there are situations in which aberrant immune tolerance can occur. For example, in autoimmune conditions such as systemic lupus erythematosus (SLE) the body does not discriminate correctly between self and non-self antigens, i.e. there is incorrect or aberrant immune tolerance, in that the individual lacks immune tolerance to their own self antigens.

Induced tolerance is tolerance to external antigens that has been created by deliberately manipulating the immune system. It is importance, for example, to protect from unpleasant or dangerous allergic reactions to such things as food (e.g. peanuts), insect stings and grass pollen (hay fever).

In addition, induced tolerance is important to enable transplanted organs (e.g., kidney, heart, liver) to survive in their new host; that is, to avoid graft rejection.

Immunological tolerance is not simply a failure to recognize an antigen; it is an active response to a particular epitope and is just as specific as an immune response.

Thus, when immune tolerance does not function correctly this can have pathological consequences.

In this regard, it has been shown to be possible to induce immunological tolerance towards particular peptide epitopes by administration of peptide epitopes in soluble form. In a normal adaptive immune response, T lymphocytes recognise internal epitopes of a protein antigen. Antigen presenting cells (APC) take up protein antigens and degrade them into short peptide fragments.

A peptide may bind to a major histocompatability complex (MHC) class I or II molecule inside the cell and be carried to the cell surface. When presented at the cell surface in conjunction with an MHC molecule, the peptide may be recognised by a T cell (via the T cell receptor (TCR)), in which case the peptide is a T cell epitope. Such epitopes in peptide form can be used to induce immunological tolerance.

Indeed, administration of soluble peptide antigens has been demonstrated as an effective means of inhibiting disease in experimental autoimmune encephalomyelitis (EAE - a model for multiple sclerosis (MS)) (Metzler and Wraith (1993) Int. Immunol. 5:1159-1165; Liu and Wraith (1995) Int. Immunol. 7:1255-1263; Anderton and Wraith (1998) Eur. J. Immunol. 28:1251-1261); and experimental models of arthritis, diabetes, and uveoretinitis (reviewed in Anderton and Wraith (1998) as above). This has also been demonstrated as a means of treating an ongoing disease in EAE (Anderton and Wraith (1998) as above).

The use of tolerogenic peptides to treat or prevent disease has attracted considerable attention. One reason for this is that it has been shown that certain tolerogenic epitopes can down-regulate responses of T cells for distinct antigens within the same tissue. This phenomenon, known as "bystander suppression" means that it should be possible to induce tolerance to more than one epitope (preferably all epitopes) within a given antigen, and to more than one antigen for a given disease, using a particular tolerogenic peptide (Anderton and Wraith (1998) as above). This obviates the need to identify all of the pathogenic antigens within a particular disease.

Peptides are also a favourable option for therapy because of their relatively low cost and the fact that peptide analogues can be produced with altered immunological properties. Peptides may thus be modified to alter their interactions with either MHC or TCR.

Streeter et al. (Streeter et al. 2015 Neurology: Neuroimmunology & Neuroinflammation, vol. 2, no. 2, e93) tested the efficacy of ATX-MS-1467 in a relevant preclinical model and assessed its safety for the treatment of patients with secondary progressive multiple sclerosis (SPMS).

ES2370957T3 relates to a composition which comprises the following myelin basic protein peptides: MBP 30-44; MBP 83-99; MBP 131-145; and MBP 140-154. The composition may be used to treat a disease, in particular multiple sclerosis and/or optical neuritis and the invention also relates to such uses and methods.

WO03/064464A1 provided a peptide which is capable of binding to an MHC class I or II molecule without further processing (i.e. an apitope) which comprises a portion of the region (131-158) of myelin basic protein.

AU2006203165A1 disclosed a method for selecting a tolerogenic peptide by selecting a peptide which is capable of binding to an MHC class I or II molecule without further processing.

WO02/16410A2 provided a method for selecting a tolerogenic peptide by selecting a peptide which is capable of binding to an MHC class I or II molecule without further processing.

### SUMMARY OF THE INVENTION

The present invention relates to a particular dosage regimen for a tolerogenic peptide or combination of tolerogenic peptides which may be used in the treatment or prevention of a disease or condition associated with aberrant hypersensitivity or pathological immune responses to endogenous or exogenous proteins that results in a loss of immune tolerance., when administered in the specific doses and at the specific times herein described.

The present inventors surprisingly found that a particular slower dose escalation protocol showed greater efficacy than a shorter/faster dose escalation protocol. As discussed in the present Examples, a slower dose escalation protocol involving four administrations over a period of eight weeks before the highest dose was administered led to greater efficacy (overall 78% reduction in the number of new lesions) than a shorter dose escalation protocol involving two administrations over a period of four weeks before the highest dose was administered (overall 54% reduction in the number of new lesions).

Furthermore, the inventors surprisingly found that extending the administration of the highest dose lead to improved prolonged suppression of new lesions after the final dose, i.e. efficacy is prolonged after the final dose is given.

The invention thus involves a combination of the longer dose escalation protocol in combination with extended administration of the highest dose.

The present invention therefore provides a tolerogenic peptide for use in the treatment or prevention in a subject of a condition associated with aberrant or pathological immune tolerance, wherein
(a) the condition is multiple sclerosis or optic neuritis, and the tolerogenic peptide is selected from myelin basic protein (MBP) peptides MBP 30-44 (SEQ ID NO: 1), MBP 83-99 (SEQ ID NO: 2), MBP 131-145 (SEQ ID NO: 3) and MBP 140-154 (SEQ ID NO: 4); or
(b) the condition is Graves' Disease, and the tolerogenic peptide is selected from thyroid stimulating hormone receptor (TSHR) peptides RNB-5D-K1 (SEQ ID NO: 5) and RNB-9B (SEQ ID NO: 6); and
and wherein the tolerogenic peptide is administered in the following doses:
Day 1: a first dose of about 15 to about 40µg;
Day 14±7 days: a second dose of about 35-65µg;
Day 28±7 days: a third dose of about 80-120µg;
Day 42±7 days : a fourth dose of about 300-500µg;
Day 56±7 days: a fifth dose of about 300-1800µg;
Day 70±7 days: a sixth dose of about 300-1800µg;
Day 84±7 days: a seventh dose of about 300-1800µg;
Day 98±7 days: an eighth dose of about 300-1800µg;
Day 112±7 days: a ninth dose of about 300-1800µg; and
Day 126±7 days: a tenth dose of about 300-1800µg.

In one aspect of the invention as described herein the fifth to tenth doses may be about 600 -1500 µg.

The reference to "±7 days" is intended to refer to the stated day (i.e. the stated day following the first administration of the peptide, which is taken as day 1) wherein the administration can occur either up to and including seven days before, or up to and including seven days after, the stated day. As such, the administration may take place 7, 6, 5, 4, 3, 2, or 1 days before, or 1, 2, 3, 4, 5, 6 or 7 days after the stated day.

In one aspect of the invention described herein the reference to "±7 days" is preferably ±3 days. That is to say the administration may take place either up to and including three days before, or up to and including three days after the stated day. As such, the administration may take place 3, 2, or 1 days before, or 1, 2, or 3 days after the stated day.

In a preferred embodiment of the invention, the tolerogenic peptide is administered in the following doses:
Day 1: a first dose of about 25µg;
Day 14: a second dose of about 50µg;
Day 28: a third dose of about 100µg;
Day 42: a fourth dose of about 400µg;
Day 56: a fifth dose of about 800 µg;
Day 70: a sixth dose of about 800 µg;
Day 84: a seventh dose of about 800 µg;
Day 98: a eighth dose of about 800 µg;
Day 112: a ninth dose of about 800 µg; and
Day 126: a tenth dose of about 800 µg.

In one aspect the fifth to tenth doses may alternatively be about 400µg.

In an alternative aspect the fifth to tenth doses may alternatively be about 1600µg.

As such, in one embodiment of the invention, the tolerogenic peptide is administered in the following doses:
Day 1: a first dose of about 25µg;
Day 14: a second dose of about 50µg;
Day 28: a third dose of about 100µg;
Day 42: a fourth dose of about 400µg;
Day 56: a fifth dose of about 400 µg;
Day 70: a sixth dose of about 400 µg;
Day 84: a seventh dose of about 400 µg;
Day 98: a eighth dose of about 400 µg;
Day 112: a ninth dose of about 400 µg; and
Day 126: a tenth dose of about 400 µg.

In a further embodiment of the invention, the tolerogenic peptide is administered in the following doses:
Day 1: a first dose of about 25µg;
Day 14: a second dose of about 50µg;
Day 28: a third dose of about 100µg;
Day 42: a fourth dose of about 400µg;
Day 56: a fifth dose of about 1600 µg;
Day 70: a sixth dose of about 1600 µg;
Day 84: a seventh dose of about 1600 µg;
Day 98: a eighth dose of about 1600 µg;
Day 112: a ninth dose of about 1600 µg; and
Day 126: a tenth dose of about 1600 µg.

Any of the teachings herein regarding the "method" of the invention are equally applicable to the "uses" encompassed by the invention.

In a further aspect of the invention as described herein an eleventh dose of about 300-1800 µg, preferably about 600-1500µg, preferably about 1200µg, more preferably about 400, 800 or 1600 µg, is administered on day 140 ±7 days, preferably on day 140±3 days, more preferably on day 140. In a preferred aspect the dose is about 800 µg.

In an even more preferred aspect a twelfth dose of about 300-1800 µg, preferably about 600-1500µg, preferably about 1200µg, more preferably about 400, 800 or 1600µg, is administered on day 154±7 days, preferably on day 154±3 days, more preferably on day 154. In a preferred aspect the dose is about 800 µg.

In a further preferred aspect a thirteenth dose of about 300-1800 µg, preferably about 600-1500µg, preferably about 1200µg, more preferably about 400, 800 or 1600µg, is administered on day 168±7 days, preferably on day 168±3 days, more preferably on day 168. In a preferred aspect the dose is about 800 µg.

In a further preferred aspect a fourteenth dose of about 300-1800 µg, preferably about 600-1500µg, preferably about 1200µg, more preferably about 400, 800 or 1600µg, is administered on day 182±7 days, preferably on day 182±3 days, more preferably on day 182. In a preferred aspect the dose is about 800 µg.

Additional doses as described above may be administered as required for a period of, for example, one month to twenty years, for example for a period of one month, two months, three months, four months, five months, six months, seven months, eight months, nine months, ten months, eleven months, one year, two years, three years, four years, five years, six years, seven years, eight years, nine years, ten years, eleven years, twelve years, thirteen years, fourteen years, fifteen years, sixteen years, seventeen years, eighteen years, nineteen years or twenty years.

### BRIEF DESCRIPTION OF THE FIGURES

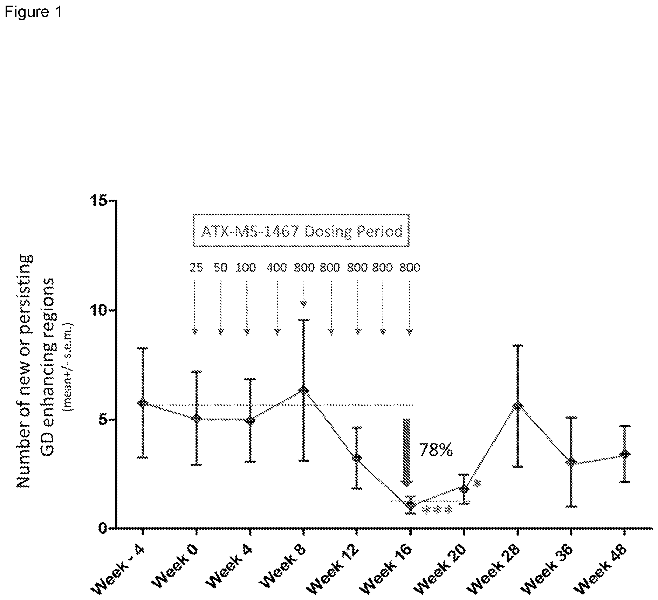

Figure 1 shows the number of new Gd lesions measured by MRI significantly reduced by ATX-MS-1467 treatment in a Phase IB clinical trial (n= 21). The dosing protocol for ATX-MS-1467 is also shown (µg).

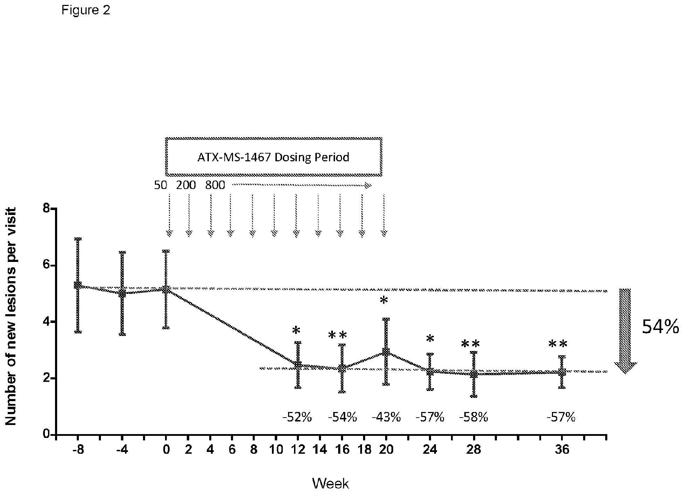

Figure 2 shows the effect of ATX-MS-1467 on new Gd enhancing lesions measured by MRI in a Phase IIA clinical trial (n=17). The dosing protocol for ATX-MS-1467 is also shown (µg). Means and SEMs were back-transformed. SEMs were derived from the statistical model. Analysis was done by generalized linear mixed model with visit as a fixed factor, patient as the subject. P values are unadjusted Wald tests after fitting this model. *p<0.05, **p<0.01, ***p<0.001.

### DETAILED DESCRIPTION OF INVENTION

In a first aspect, the present invention relates to a tolerogenic peptide for use in the treatment or prevention in a subject of a condition associated with aberrant or pathological immune tolerance, wherein
(a) the condition is multiple sclerosis or optic neuritis, and the tolerogenic peptide is selected from myelin basic protein (MBP) peptides MBP 30-44 (SEQ ID NO: 1), MBP 83-99 (SEQ ID NO: 2), MBP 131-145 (SEQ ID NO: 3) and MBP 140-154 (SEQ ID NO: 4); or
(b) the condition is Graves' Disease, and the tolerogenic peptide is selected from thyroid stimulating hormone receptor (TSHR) peptides RNB-5D-K1 (SEQ ID NO: 5) and RNB-9B (SEQ ID NO: 6); and
and wherein the tolerogenic peptide is administered in the following doses:
Day 1: a first dose of about 15 to about 40µg;
Day 14±7 days: a second dose of about 35-65µg;
Day 28±7 days: a third dose of about 80-120µg;
Day 42±7 days : a fourth dose of about 300-500µg;
Day 56±7 days: a fifth dose of about 300-1800µg;
Day 70±7 days: a sixth dose of about 300-1800µg;
Day 84±7 days: a seventh dose of about 300-1800µg;
Day 98±7 days: an eighth dose of about 300-1800µg;
Day 112±7 days: a ninth dose of about 300-1800µg; and
Day 126±7 days: a tenth dose of about 300-1800µg.

In one aspect of the invention as described herein the fifth to tenth doses may be about 600 -1500 µg.

As discussed above and herein, the dosage regimen according to the invention maximises efficacy of a dose escalation protocol. The dosage regimen according to the invention improves the efficacy and prolongs the efficacy compared with a dosage regimen in which a shorter dose escalation is used.

The present Examples show that following the final dose, the dosage regimen according to the present invention prolongs the time or duration of the observed decrease in clinical effects, for example Gd lesion formation. In one aspect of the invention as described herein the dosage regimen is for prolonging the time or duration of a decrease in clinical effects. In one aspect a decrease in formation of Gd lesions may be prolonged.

### TOLEROGENIC PEPTIDES AND TOLERANCE

As used herein, the term "tolerogenic" means capable of inducing tolerance to a particular antigen.

Tolerance is the failure to respond to an antigen. Tolerance to self antigens is an essential feature of the immune system, when this is lost, autoimmune disease can result. The adaptive immune system must maintain the capacity to respond to an enormous variety of infectious agents while avoiding autoimmune attack of the self antigens contained within its own tissues. This is controlled to a large extent by the sensitivity of immature T lymphocytes to apoptotic cell death in the thymus (central tolerance). However, not all self antigens are detected in the thymus, so death of self-reactive thymocytes remains incomplete, which can lead to, for example, autoimmune disorders.

There are also mechanisms by which tolerance may be acquired by mature self-reactive T lymphocytes in the peripheral tissues (peripheral tolerance). A review of the mechanisms of central and peripheral tolerance is given in Anderton et al. (1999) (Immunological Reviews 169:123-137).

Tolerance may result from or be characterised by the induction of anergy in at least a portion of CD4+ T cells. In order to activate a T cell, a peptide must associate with a "professional" APC capable of delivering two signals to T cells. The first signal (signal 1) is delivered by the MHC-peptide complex on the cell surface of the APC and is received by the T cell via the TCR. The second signal (signal 2) is delivered by costimulatory molecules on the surface of the APC, such as CD80 and CD86, and received by CD28 on the surface of the T cell. It is hypothesized that when a T cell receives signal 1 in the absence of signal 2, it is not activated and, in fact, becomes anergic. Anergic T cells are refractory to subsequent antigenic challenge, and may be capable of suppressing other immune responses. Anergic T cells are thought to be involved in mediating T cell tolerance.

### APITOPES ARE TOLEROGENIC PEPTIDES

In one aspect of the invention, the tolerogenic peptide is an apitope.

As discussed above, T lymphocytes are capable of recognising internal epitopes of a protein antigen. Antigen presenting cells (APC) take up protein antigens and degrade them into short peptide fragments. A peptide may bind to a major histocompatibility complex (MHC) molecule inside the cell and be carried to the cell surface. When presented at the cell surface in conjunction with an MHC molecule, the peptide may be recognised by a T cell (via the T cell receptor (TCR)), in which case the peptide is a T cell epitope.

The present inventors have shown previously that if a peptide epitope is of an appropriate size to be presented by immature APC without antigen processing, it can induce immunological tolerance (International patent publication number WO02/16410).

The epitopes which do not require further processing are capable of inducing tolerance, and have been termed "apitopes" (Antigen Processing Independent epiTOPES) by the inventors.

### ANTIGEN PROCESSING INDEPENDENT PRESENTATION SYSTEMS (APIPS)

Tolerogenic peptides or apitopes are therefore capable of binding to an MHC molecule *in vitro* and being presented to a T cell without antigen processing.

It is possible to test whether a peptide is capable of binding to an MHC molecule without antigen processing using a "processing free" system. Such a system should be capable of presenting antigen via MHC molecules to T cells, but incapable of processing antigen. Thus peptides may be tested for their capacity to bind to an MHC molecule *in vitro* and being presented to a T cell without antigen processing using an antigen processing independent presentation system (APIPS).

Examples of APIPS include:
a) fixed APC (with or without antibodies to CD28);
b) Lipid membranes containing Class I or II MHC molecules (with or without antibodies to CD28); and
c) purified natural or recombinant MHC in plate-bound form (with or without antibodies to CD28).

It is known to use fixed APC to investigate T cell responses, for example in studies to investigate the minimal epitope within a polypeptide, by measuring the response to truncated peptides (Fairchild et al (1996) Int. Immunol. 8:1035-1043). APC may be fixed using, for example formaldehyde (usually paraformaldehyde) or glutaraldehyde.

Lipid membranes (which may be planar membranes or liposomes) may be prepared using artificial lipids or may be plasma membrane/microsomal fractions from APC.

In use, the APIPS may be applied to the wells of a tissue culture plate. Peptide antigens are then added and binding of the peptide to the MHC portion of the APIPS is detected by addition of selected T cell lines or clones. Activation of the T cell line or clone may be measured by any of the methods known in the art, for example via ³H-thymidine incorporation or cytokine secretion.

If a peptide is capable of being presented to a T cell by an APIPS, then it is capable of binding to the MHC molecule without antigen processing, and is an apitope.

Without wishing to be bound by theory, it is hypothesized that peptides which require processing before they can be presented in conjunction with MHC molecules do not induce tolerance because they have to be handled by mature antigen presenting cells. Mature antigen presenting cells (such as macrophages, B cells and dendritic cells) are capable of antigen processing, but also of delivering both signals 1 and 2 to a T cell, leading to T cell activation. Apitopes, on the other hand, will be able to bind class II MHC on immature APC. Thus they will be presented to T cells without costimulation, leading to T cell anergy and tolerance.

Of course, apitopes are also capable of binding to MHC molecules at the cell surface of mature APC. However, the immune system contains a greater abundance of immature than mature APC (it has been suggested that less than 10% of dendritic cells are activated, Summers et al. (2001) Am. J. Pathol. 159: 285-295). The default position to an apitope will therefore be anergy/tolerance, rather than activation.

It has been shown that, when tolerance is induced by peptide inhalation, or intradermal or subcutaneous administration, the capacity of antigen-specific CD4+ T cells to proliferate is reduced. Also, the production of IL-2, IFN-y and IL-4 production by these cells is downregulated, but production of IL-10 is increased. Neutralisation of IL-10 in mice in a state of peptide-induced tolerance has been shown to restore completely susceptibility to disease. It has been proposed that a population of regulatory cells persist in the tolerant state which produce IL-10 and mediate immune regulation (Burkhart et al (1999) Int. Immunol. 11:1625-1634).

The induction of tolerance can therefore be monitored by various techniques including:
(a) reduced susceptibility to contract the disease for which the peptide is a target epitope *in vivo*;
(b) the induction of anergy in CD4+ T cells (which can be detected by subsequent challenge with antigen *in vitro*);
(c) changes in the CD4+ T cell population, including
   (i) reduction in proliferation;
   (ii) down-regulation in the production of IL-2, IFN-y and IL-4; and
   (iii) increase in the production of L - 10.

### IDENTIFICATION OF PEPTIDES CONTAINING T CELL EPITOPES

There are a number of methods known in the art to identify the T cell epitopes within a given antigen.

Naturally processed epitopes may be identified by mass spectrophotometric analysis of peptides eluted from antigen-loaded APC. These are APC that have either been encouraged to take up antigen, or have been forced to produce the protein intracellularly by transformation with the appropriate gene. Typically APC are incubated with protein either in solution or suitably targeted to the APC cell surface. After incubation at 37°C the cells are lysed in detergent and the class II protein purified by, for example affinity chromatography.

Treatment of the purified MHC with a suitable chemical medium (for example, acid conditions) results in the elution of peptides from the MHC. This pool of peptides is separated and the profile compared with peptide from control APC treated in the same way. The peaks unique to the protein expressing/fed cells are analysed (for example by mass spectrometry) and the peptide fragments identified. This procedure usually generates information about the range of peptides (usually found in "nested sets") generated from a particular antigen by antigen processing.

Another method for identifying epitopes is to screen a synthetic library of peptides which overlap and span the length of the antigen in an in vitro assay. For example, peptides which are 15 amino acids in length and which overlap by 5 or 10 amino acids may be used. The peptides are tested in an antigen presentation system which comprises antigen presenting cells and T cells. For example, the antigen presentation system may be a murine splenocyte preparation, a preparation of human cells from tonsil or PBMC. Alternatively, the antigen presentation system may comprise a particular T cell line/clone and/or a particular antigen presenting cell type.

T cell activation may be measured via T cell proliferation (for example using H-thymidine incorporation) or cytokine production. Activation of TH-type CD4+ T cells can, for example be detected via IFNy production which may be detected by standard techniques, such as an ELISPOT assay.

Overlapping peptide studies usually indicate the area of the antigen in which an epitope is located. The minimal epitope for a particular T cell can then be assessed by measuring the response to truncated peptides. For example if a response is obtained to the peptide comprising residues 1-15 in the overlapping library, sets which are truncated at both ends (i.e. 1-14, 1-13, 1-12 etc. and 2-15, 3-15, 4-15 etc.) can be used to identify the minimal epitope.

### PEPTIDES

The term "peptide" is used in the normal sense to mean a series of residues, typically L-amino acids, connected one to the other typically by peptide bonds between the α-amino and carboxyl groups of adjacent amino acids The term includes modified peptides and synthetic peptide analogues.

A peptide for use according to the present invention may be any length that is capable of binding to an MHC molecule without further processing.

Peptides that bind to MHC class I molecules are typically 7 to 13, more usually 8 to 10 amino acids in length. The binding of the peptide is stabilised at its two ends by contacts between atoms in the main chain of the peptide and invariant sites in the peptide-binding groove of all MHC class I molecules. There are invariant sites at both ends of the groove which bind the amino and carboxy termini of the peptide. Variations in peptide length are accommodated by a kinking in the peptide backbone, often at proline or glycine residues that allow the required flexibility.

Peptides which bind to MHC class II molecules are typically between 8 and 20 amino acids in length, more usually between 10 and 17 amino acids in length, and can be much longer. These peptides lie in an extended conformation along the MHC II peptide-binding groove which (unlike the MHC class I peptide-binding groove) is open at both ends. The peptide is held in place mainly by main-chain atom contacts with conserved residues that line the peptide-binding groove. In one aspect the peptide may be between 9 and 30 amino acids long.

The peptide for use according to the present invention may be made using chemical methods (Peptide Chemistry, A practical Textbook. Mikos Bodansky, Springer- Verlag, Berlin.). For example, peptides can be synthesized by solid phase techniques (Roberge JY et al (1995) Science 269: 202-204), cleaved from the resin, and purified by preparative high performance liquid chromatography (e.g., Creighton (1983) Proteins Structures And Molecular Principles, WH Freeman and Co, New York NY). Automated synthesis may be achieved, for example, using the ABI 43 1 A Peptide Synthesizer (Perkin Elmer) in accordance with the instructions provided by the manufacturer.

The peptide may alternatively be made by recombinant means, or by cleavage from a longer polypeptide. For example, the peptide may be obtained by cleavage from the target antigen. The composition of a peptide may be confirmed by amino acid analysis or sequencing (e.g., the Edman degradation procedure).

In a preferred embodiment the peptide is derivable from a target antigen. A target antigen is a molecule (for example a protein or glycoprotein) which is processed by APC and recognised by T cells during the course of the disease. The target antigen will, of course, depend on the target disease. Preferably the peptide is derivable from a fragment of the antigen which arises by natural processing of the antigen by an APC.

Examples of tolerogenic peptides are known in the art. For example, WO2009/071886, WO2014/072958 and WO2010/133834 relate to tolerogenic peptides derivable from the factor VIII protein, which have a utility in the treatment of haemophilia.

In one aspect, the subject may have multiple sclerosis or optic neuritis, and accordingly the tolerogenic peptide may be derived from myelin basic protein. Myelin basic protein (MBP) is an 18.5 kDa protein isolatable from human brain white matter. The mature protein has 170 amino acids and the sequence is widely available in the literature (see for example: Chou et al (1986) J. Neurochem. 46:47-53, Figure 1; Kamholz et al (1986), PNAS 83:4962-4966, Figure 2; US Patent No. 5,817,629, SEQ ID NO: 1; Roth et al (1987), J. Neurosci. Res. 17:321-328, Figure 4; Medeveczky et al (2006), FEBS Letters 580:545-552, Figure 3B).

In particular, the tolerogenic peptide may be selected from:
MBP 30-44: H-Pro-Arg-His-Arg-Asp-Thr-Gly-Ile-Leu-Asp-Ser-Ile-Gly-Arg-Phe-NH2 (SEQ ID NO:1)
MBP 83-99: H-Glu-Asn-Pro-Val-Val-His-Phe-Phe-Lys-Asn-Ile-Val-Thr-Pro-Arg-Thr-Pro-NH2 (SEQ ID NO:2)
MBP 131-145: H-Ala-Ser-Asp-Tyr-Lys-Ser-Ala-His-Lys-Gly-Phe-Lys-Gly-Val-Asp-NH2 (SEQ ID NO:3); and
MBP 140-154: H-Gly-Phe-Lys-Gly- Val-Asp-Ala-Gln-Gly- Thr-Leu-Ser-Lys-Ile-Phe-N H2 (SEQ ID NO:4).

These peptides are also described in WO2009/056833.

MBP 30-44 is also referred to as ATX-MS-01; MBP83-99 is also referred to as ATX-MS-04; MBP 131-145 is also referred to as ATX-MS-06; and MBP 140-154 is also referred to as ATX-MS-07.

In a preferred aspect of the invention as described herein, all four of MBP 30-44, 83-99, 131-145 and 140-154 are administered to the subject according to the dosage regimen of the invention as described herein. The combination of all four peptides is referred to herein as "ATX-MS-1467" in some instances.

In one aspect a composition comprising the four peptides is administered to a subject according to the present invention.

In one aspect, the subject may have Graves' Disease, and accordingly the tolerogenic peptide may be derived from the thyroid stimulating hormone receptor (TSHR).

TSHR is a G-protein coupled receptor on thyroid follicular cells in the thyroid gland that stimulates the production of thyroxine (T4) and triiodothyronine (T3) via a cAMP signal cascade upon binding of its ligand, the thyroid-stimulating hormone (TSH). Upon internalization, degradation and presentation of the TSHR by APCs, T cells become activated and interact with auto-reactive B cells, which in turn produce stimulating agonistic auto-antibodies directed against TSHR. The thyroid-stimulating immunoglobulins bind to the same receptor pocket as the TSH, activating the TSHR mediated signal transduction and leading to the production of excess thyroid hormone from the thyroid gland and thyroid growth.

Thus, in one aspect, the tolerogenic peptide may be selected from:
KKKKYVSIDVTLQQLESHKKK (SEQ ID No 5)
GLKMFPDLTKVYSTD (SEQ ID No 6)
KKKKYVSIDVTLQQLESHKKK (SEQ ID NO: 5) is also referred to as RNB-5D-K1 herein. GLKMFPDLTKVYSTD (SEQ ID NO: 6) is also referred to as RNB-9B herein.

In one aspect a composition comprising a peptide of both SEQ ID NO:5 and SEQ ID NO:6 is administered to a subject according to the present invention.

In the composition, the relative ratio of the peptides (MBP 30-44:MBP 83-99:MBP 131-145:MBP 140-154 or RNB-5D-K1: RNB-9B) may be approximately 1:1:1:1 or 1:1, respectively (by weight or moles). Alternatively the relative ratios of each peptide may be altered, for example, to focus the tolerogenic response on a particular sub-set of autoreactive T-cells or if it is found that one peptide works better than the others in particular HLA types.

The terms "MBP 30-44", "MBP 83-99", "MBP 131-145", "MBP 140-154", RNB-5D-K1 and RNB-9B encompasses modified peptides. For example the peptides may be mutated, by amino acid insertion, deletion or substitution, so long as the MHC binding-specificity of the unmodified peptide is retained, together with its capacity to be presented to a T cell. The peptide may, for example, have 5, 4, 3, 2, 1 or 0 mutations from the unmodified sequence.

Modifications may be made without changing the amino acid sequence of a peptide. For example, D-amino acids or other unnatural amino acids can be included, the normal amide bond can be replaced by ester or alkyl backbone bonds, N-or C-alkyl substituents, side chain modifications, and constraints such as disulphide bridges and side chain amide or ester linkages can be included. Such changes may result in greater in vivo stability of the peptide, and a longer biological lifetime.

Modification of epitopes may be performed based on predictions for more efficient T-cell induction derived using the program "Peptide Binding Predictions" devised by K. Parker (NIH) which may be found at http://www-bimas.dcrt.nih.gov/cgi-bin/molbio/ken_parker_comboform (see also Parker, K. C et al. 1994.J.Immunol. 152:163).

Described herein are peptides comprising or consisting of an amino acid sequence having at least 60%, 61%, 62%, 63%, 64%, 65%, 66%, 67%, 68%, 69%, 70%, 71%, 72%, 73%, 74%, 75%, 76%, 77%, 78%, 79%, 70%, 81%, 82%, 83%, 84, 85%, 86%, 87%, 88%, 89%, 90%, 91%, 92%, 93%, 94%, 95%, 96%, 97%, 98%, 99% or 100% identity with a peptide of any of SEQ ID NOs: 1 to 6. In one aspect the peptide has at least 70%, 75%, 80%, 85%, 90%, 95% or 100% sequence identity to any of SEQ ID NOs:1 to 6.

Sequence identity may be assessed by any convenient method. However, for determining the degree of sequence identity between sequences, computer programs that make multiple alignments of sequences are useful, for instance Clustal W (Thompson et al., (1994) Nucleic Acids Res., 22: 4673-4680). Programs that compare and align pairs of sequences, like ALIGN (Myers ef a/., (1988) CABIOS, 4: 1-17), FASTA (Pearson er a/., (1988) PNAS, 85:2444-2448; Pearson (1990), Methods Enzymol., 183: 63-98) and gapped BLAST (Altschul er a/., (1997) Nucleic Acids Res., 25: 3389-3402) are also useful for this purpose. Furthermore, the Dali server at the European Bioinformatics institute offers structure-based alignments of protein sequences (Holm (1993) J. Mol. Biol., 233: 123-38; Holm (1995) Trends Biochem. Sci., 20: 478-480; Holm (1998) Nucleic Acid Res., 26: 316-9).

Multiple sequence alignments and percent identity calculations may be determined using the standard BLAST parameters, (using sequences from all organisms available, matrix Blosum 62, gap costs: existence 11, extension 1).

Alternatively, the following program and parameters may be used: Program: Align Plus 4, version 4.10 (Sci Ed Central Clone Manager Professional Suite). DNA comparison: Global comparison, Standard Linear Scoring matrix, Mismatch penalty = 2, Open gap penalty = 4, Extend gap penalty = 1. Amino acid comparison: Global comparison, BLOSUM 62 Scoring matrix.

Variants of the stated or given sequences retain the functional activity of the parent i.e. the variants are functionally equivalent, in other words they have or exhibit an activity of the parent peptide as defined herein. Such variants may comprise amino acid substitutions, additions or deletions (including truncations at one or both ends) of the parent sequence e.g. of one or more e.g. 1 to 14 amino acids.

Also described herein are functionally-equivalent derivatives in which one or more of the amino acids are chemically derivitised, e.g. substituted with a chemical group.

Thus, peptides described herein can comprise parts or fragments of SEQ ID NOs:1-6, provided that the peptide retains the required activity. Fragments or parts of SEQ ID NOs:1-6 may for example be from 6 to 14 residues in length, e.g. 6, 7, 8, 9, 10, 11, 12 or 13 residues in length.

Peptides described herein may comprise between 8 and 30 amino acids, for example 8 to 25 amino acids, 8 to 20 amino acids, 8 to 15 amino acids or 8 to 12 amino acids. A peptide may thus be 8, 9, 10, 11, 12, 13, 14, 15, 16, 17, 18, 19, 20, 21, 22, 23, 24, 25, 26, 27, 28, 29 or 30 amino acids in length.

The peptides may be formulated into the composition as neutral or salt forms. Pharmaceutically acceptable salts include the acid addition salts (formed with free amino groups of the peptide) and which are formed with inorganic acids such as, for example, hydrochloric or phosphoric acids, or such organic acids such as acetic, oxalic, tartaric and maleic. Salts formed with the free carboxyl groups may also be derived from inorganic bases such as, for example, sodium, potassium, ammonium, calcium, or ferric hydroxides, and such organic bases as isopropylamine, trimethylamine, 2 ethylamino ethanol, histidine and procaine.

WO2015/019302 relates to tolerogenic peptides derivable from human thyroid stimulating hormone receptor (TSHR) which have a utility in the treatment of Graves' Disease. Peptides described in WO2015/019302 are suitable for use according to the present invention. Peptides described in WO2016/103213 are also suitable for use according to the present invention.

### TARGET DISEASES

Peptides as described herein are used according to the present invention to treat or prevent a condition associated with aberrant or pathological immune tolerance. As discussed above, immune tolerance is a state of unresponsiveness of the immune system to substances or tissue that have the capacity to elicit an immune response

Apitopes for MHC class I may be used, for example, to modify anti-viral CD8+ responses in a tolerogenic fashion.

An apitope for MHC class II is likely to be particularly useful in diseases which are mediated by CD4+ T cell responses. For example, diseases which are established or maintained by an inappropriate or excessive CD4+T cell response.

Such a peptide is likely to be particularly useful in the treatment of hypersensitivity disorders. Hypersensitivity reactions include:
(i) allergies, resulting from inappropriate responses to innocuous foreign substances;
(ii) autoimmune diseases, resulting from responses to self tissue antigens by autoreactive T cells; and
(iii) graft rejection, resulting from responses to a transplant.

Examples of allergies include, but are not limited to: hay fever, extrinsic asthma, insect bite and sting allergies, food and drug allergies, allergic rhinitis, bronchial asthma chronic bronchitis, anaphylactic syndrome, urticaria, angioedema, atopic dermatitis, allergic contact dermatitis, erythema nodosum, erythema multiforme, Stevens- Johnson Syndrome, rhinoconjunctivitis, conjunctivitis, cutaneous necrotizing venulitis, inflammatory lung disease and bullous skin diseases.

Examples of the autoimmune diseases include, but are not limited to: rheumatoid arthritis (RA), myasthenia gravis (MG), multiple sclerosis (MS), systemic lupus erythematosus (SLE), autoimmune thyroiditis (Hashimoto's thyroiditis), Graves' disease, uveitis (including intermediate uveitis) inflammatory bowel disease, autoimmune uveoretinitis, polymyositis and certain types of diabetes, systemic vasculitis, polymyositis-dermatomyositis, systemic sclerosis (scleroderma), Sjogren's Syndrome, ankylosing spondylitis and related spondyloarthropathies, rheumatic fever, hypersensitivity pneumonitis, allergic bronchopulmonary aspergillosis, inorganic dust pneumoconioses, sarcoidosis, autoimmune hemolytic anemia, immunological platelet disorders, cryopathies such as cryofibrinogenemia and autoimmune polyendocrinopathies.

In a preferred embodiment of the invention the subject has multiple sclerosis and/or optic neuritis. In a preferred aspect the subject preferably has multiple sclerosis.

In an alternative preferred embodiment of the invention the subject has Graves' Disease.

A variety of tissues are commonly transplanted in clinical medicine, including kidney, liver, heart lung, skin, cornea and bone marrow. All grafts except corneal and some bone marrow grafts usually require long-term immunosuppression at present.

### MULTIPLE SCLEROSIS

Multiple sclerosis (MS) is a chronic inflammatory disease characterised by multiple demyelinating lesions disseminated throughout the CNS white matter and occurring at various sites and times (McFarlin and McFarland, 1982 New England J. Medicine 307:1183-1188 and 1246-1251).

Gradual destruction of myelin occurs in patches throughout the brain and/ or spinal cord, interfering with neural connectivity and causing muscular weakness, loss of coordination and speech and visual disturbances.

References to multiple sclerosis or MS herein are intended to encompass the four types of multiple sclerosis, including: relapsing-remitting, primary-progressive, secondary-progressive, and progressive-relapsing.

Relapsing-remitting MS is the most common form and affects about 85 percent of those first diagnosed with the disease and affects around 60 percent of people living with MS. While most new diagnoses of multiple sclerosis are categorized as relapsing-remitting, an individual may develop another form of the disease over time.

In relapsing-remitting multiple sclerosis, patients experience a period of active symptoms, called relapsing, followed by periods that are symptom-free, called remitting. Some people with relapsing-remitting multiple sclerosis go a year or more between relapses; others have them more frequently.

As regards secondary-progressive MS, relapsing-remitting patients often develop secondary-progressive multiple sclerosis somewhere between 10 and 15 years after their initial diagnosis of relapsing-remitting multiple sclerosis. Once diagnosed with secondary-progressive multiple sclerosis, people will notice a change in the pattern of their disease. While some acute attacks (exacerbations) and periods of remission may still occur, they happen less frequently, recovery is less complete, and symptoms become chronic, gradually worsening over time.

Approximately 10 percent of MS patients are diagnosed with primary-progressive multiple sclerosis. In this more serious form of multiple sclerosis, the exacerbations, or attacks, seen in people with relapsing-remitting MS are rare, if they occur at all. Instead, MS symptoms worsen over time, gradually leading to disability.

Progressive-relapsing MS is the least common type of multiple sclerosis. Like primary-progressive MS, this form of multiple sclerosis is characterized by a gradual worsening of symptoms over time, but patients with this type of MS will also experience exacerbations and remissions. Unlike relapsing-remitting MS, however, people with progressive-relapsing multiple sclerosis do not typically regain complete functioning after a symptom relapse. In progressive-relapsing MS, disability is caused by the combination of disease progression and incomplete recovery after an attack.

In one aspect of the invention as described herein the subject has relapsing MS. Reference to "relapsing MS" encompasses patients with relapsing-remitting MS, as well as patients that are moving from relapsing-remitting MS to secondary progressive MS.

### OPTIC NEURITIS

Optic Neuritis (ON) is an inflammation, with accompanying demyelination, of the optic nerve serving the retina of the eye. It is a variable condition and can present with any of the following symptoms: blurring of vision, loss of visual acuity, loss of some or all colour vision, complete or partial blindness and pain behind the eye.

Optic Neuritis is one of the most frequently presenting symptoms of multiple sclerosis and is the most common symptom at onset of MS. ON can, however, be attributable to causes other than MS, such as ischemic optic neuropathy.

ON presents unilaterally (in one eye only) in 70% of cases.

Most typically, Optic Neuritis first affects people aged between 15 and 50 years of age. In this age group, studies indicate that more than 50% of patients will convert to Multiple Sclerosis within 15 years. As with MS, women are about twice as likely as men to present with ON and the prevalence in Caucasian peoples is higher than in other racial groups.

The main symptoms of Optic Neuritis are:
- Loss of visual acuity (blurring of vision).
- Eye pain.
- Dyschromatopsia (reduced colour vision).
- Movement and sound phosphenes (visual flashing sensations brought about by side-to-side eye movement or sound).
- Uhthoff's symptom, the worsening of symptoms with heat or exhaustion.

Treatment of ON with a composition according to the present invention may prevent, reduce or ameliorate any of these symptoms. In order to monitor progression of ON, visual accuity may conveniently be measured using a Snellens chart.

### GRAVES DISEASE

In one aspect of the invention the subject has Graves' Disease.

Graves' disease is an autoimmune disease that affects the thyroid. It is characterised by an overactive thyroid gland, which results in the production of an excessive amount of thyroid hormone and enlargement of the thyroid gland (goitre). The resulting state of hyperthyroidism may cause a wide range of neuropsychological and physical symptoms. Graves' disease is the most common cause of hyperthyroidism (60-90% of all cases) and usually presents itself during midlife, but also appears in children, adolescents, and the elderly. It affects up to 2% of the female population and is between five and ten times as common in females as in males. Paediatric Graves' disease affects about 6,000 children in the United States (US) and 6,000 in the European Union (EU). Graves' disease is also the most common cause of severe hyperthyroidism, which is accompanied by more clinical signs and symptoms and laboratory abnormalities as compared with milder forms of hyperthyroidism. There is a strong hereditary component linked to Graves' disease. There are no recent population studies on Graves' disease, however, a few quasi population studies on hyperthyroidism do exist and all estimates for incidence and prevalence of Graves' disease are thus approximate. The incidence of hyperthyroidism varies from 26:100,000 to 93:100,000 and the overall prevalence is estimated to be at 1.3%, with 40% of cases being overt and 60% subclinical.

About 30-50% of people with Graves' disease will also suffer from Graves' opthalmopathy (also known as Graves' orbitopathy or thyroid eye disease) (GO), a protrusion of one or both eyes. Many cases of GO are mild and self-limiting, however 20% of cases have significant/moderate to severe disease, with at least half of these requiring steroids and 3-5% of GO patients have painful, sight-threatening disease with dysthyroid optic neuropathy. The buldging of the eyes may cause severe dryness of the cornea as the eye lids are unable to close at night. Increased pressure on the optic nerve can lead to visual field defects and vision loss. GO may also be associated with pretibial myxedemia.

The symptoms and signs of Graves' disease virtually all result from the direct and indirect effects of hyperthyroidism, with main exceptions being GO, goitre and pretibial myxedema. Symptoms of hyperthyroidism may include insomnia, hand tremor, hyperactivity, hair loss, excessive sweating, heat intolerance and weight loss despite increased appetite. Further signs are most commonly a diffusely enlarged (usually symmetric) non-tender thyroid, lid lag, excessive lacrimation due to GO, arrhythmias of the heart and hypertension. Thyrotoxic patients may experience behavioural and personality changes, such as psychosis, agitation, and depression. In milder hyperthyroidism, patients may experience less overt manifestations, for example anxiety, restlessness, irritability and emotional lability.

In a preferred embodiment of the present invention, the subject of the method is a mammal, preferably a cat, dog, horse, donkey, sheep, pig, goat, cow, mouse, rat, rabbit or guinea pig, but most preferably the subject is a human.

As defined herein "treatment" refers to reducing, alleviating or eliminating one or more symptoms of the condition which is being treated, relative to the symptoms prior to treatment.

"Prevention" (or prophylaxis) refers to delaying or preventing the onset of the symptoms of the condition. Prevention may be absolute (such that no condition occurs) or may be effective only in some individuals or for a limited amount of time.

### DOSAGE REGIMEN

The invention relates to a "dose escalation" dosing regimen wherein a plurality of doses is given to the patient in ascending concentrations.

According to the invention, the following dosage regimen is employed:
Day 1: a first dose of about 15 to about 40µg;
Day 14±7 days: a second dose of about 35-65µg;
Day 28±7 days: a third dose of about 80-120µg;
Day 42±7 days : a fourth dose of about 300-500µg;
Day 56±7 days: a fifth dose of about 300-1800µg;
Day 70±7 days: a sixth dose of about 300-1800µg;
Day 84±7 days: a seventh dose of about 300-1800µg;
Day 98±7 days: an eighth dose of about 300-1800µg;
Day 112±7 days: a ninth dose of about 300-1800µg; and
Day 126±7 days: a tenth dose of about 300-1800µg.

In one aspect of the invention as described herein the fifth to tenth doses may be about 600 -1500 µg.

Thus, on day 1 about 15 to about 40µg of the tolerogenic peptide is administered as the first dose. In a preferred embodiment about 25µg is administered, e.g. about 17 to 35, 18 to 33, 20 to 30, 22 to 28 or 24 to 26µg. In a most preferred embodiment 25µg is administered.

The second dose is administered on day 14±7, preferably 3, days, i.e. with each "day" being counted as approximately a 24 hour period following "day 1". In a preferred embodiment the second dose is administered on day 14.

Doses may be administered at the same time of day or at a different time of day. Preferably the first, second, third and subsequent doses are administered at the same time of day.

The second dose is about 35 to about 65µg of the tolerogenic peptide. In a preferred embodiment the dose is about 37 to 60, 40 to 58, 42 to 56 or 45 to 55µg. In a particularly preferred embodiment the second dose is about 50µg, e.g. 45, 46, 47, 48, 49, 51, 52, 43, 54 or 55µg. In a most preferred embodiment the second dose is 50µg

The third dose is administered on day 28±7, preferably 3, days, i.e. with each "day" being counted as a 24 hour period following the first dose on "day 1". In a preferred embodiment the third dose is administered on day 28.

The third dose is about 80 to about 120µg of the tolerogenic peptide. In a preferred embodiment the third dose is about 85 to 115, 87 to 113, 90 to 110, 92 to 107, 95 to 105 or 97 to 102µg. In a particularly preferred embodiment the third dose is about 100µg, e.g. 95, 96, 97, 98, 99, 101, 102, 103, 104, or 105µg. In a most preferred embodiment the third dose is 100µg.

The fourth dose is administered on day 42±7, preferably 3, days, i.e. with each "day" being counted as a 24 hour period following "day 1". In a preferred embodiment the third dose is administered on day 42.

The fourth dose is about 300 to about 500µg of the tolerogenic peptide. In a preferred embodiment the forth dose is about 320 to 480, 350 to 450, 365 to 440, 370 to 430, 380 to 420 or 390 to 410µg. In a particularly preferred embodiment the forth dose is about 400µg, e.g. 395, 396, 397, 398, 399, 401, 402, 403, 404, or 405µg. In a most preferred embodiment the forth dose is 400µg.

Following the fourth dose, subsequent doses of about 300 to about 1800µg of the tolerogenic peptide are administered at approximately every 14 days±7, preferably 3, days. At least 6 doses of about 300 to about 1800µg are administered. More than 6 doses of about 300 to about 1800µg may also be administered as discussed herein, for example 7, 8, 9, 10, 11, 12, 13, 14, 15, 16, 17, 18, 19 or 20 doses may be administered. The doses may be repeated for periods of time up to, for example, twenty years, as discussed above.

The intervals between the doses according to the invention may thus be approximately 14 days or two weeks. The intervals may be the same or may be different throughout the period. For example, different combinations of the intervals may be used, e.g. the first subsequent dose may be at an interval of 14 days, whereas the second subsequent dose may be administered at an interval of 15 days. In a most preferred embodiment the subsequent doses are administered every 14 days or every two weeks.

The subsequent doses, doses five to ten (and optionally doses eleven, twelve, thirteen and fourteen) are about 300 to about 1800µg of the tolerogenic peptide. In a preferred embodiment the fifth to tenth (and optionally eleventh, twelfth, thirteenth and fourteenth) dose is each about 400, 500, 600, 800, 1000, 1200 or 1600µg, for example 400 to 1600, 600 to 1200, 600 to 1000 or 600 to 800 µg. In one aspect the fifth to tenth (and optionally eleventh to fourteenth) dose is each about 350, 400, 450, 500, 550, 600, 650, 700, 750, 800, 850, 900, 950, 1000, 1050, 1100, 1150, 1200, 1250, 1300, 1350, 1400, 1450, 1500, 1550, 1600 or 1650µg. In a particularly preferred embodiment the fifth to tenth (and optionally eleventh to fourteenth) dose is each about 800µg, e.g. 785, 786, 787, 788, 789, 790, 791, 792, 793, 794, 795, 796, 797, 798, 799, 801, 802, 803, 804, 805, 806, 807, 808, 809, 810 or 815µg. In a most preferred embodiment the fifth to tenth dose is 800µg.

In an alternative embodiment the fifth to tenth (and optionally eleventh to fourteenth) doses is each about 400µg, e.g. 385, 386, 387, 388, 389, 390, 391, 392, 393, 394, 395, 396, 397, 398, 399, 401, 402, 403, 404, 405, 406, 407, 408, 409, 410 or 415µg. In a most preferred embodiment the fifth to tenth dose is 400µg.

In an alternative embodiment the fifth to tenth (and optionally eleventh to fourteenth) doses is each about 1600µg, e.g. 1585, 1586, 1587, 1588, 1589, 1590, 1591, 1592, 5193, 194, 1595, 1596, 1597, 1598, 1599, 1601, 1602, 1603, 1604, 1605, 1606, 1607, 1608, 1609, 1610 or 1615µg. In a most preferred embodiment the fifth to tenth dose is 600µg.

In alternative aspects of the invention further doses are administered after the doses discussed above, i.e. the subject may continue to receive doses after the initial doses discussed herein.

The doses can be adjusted accordingly if necessary by methods known in the art to the skilled man.

For administration of peptides described herein *in vivo*, any mode of administration common or standard in the art may be used, e.g. oral, intravenous, intramuscular, subcutaneous, sublingual, intranasal, intradermal, suppository routes or implanting.

In a preferred embodiment of the invention as described herein administration is intradermal, e.g. by intradermal injection. Intradermal administration can be carried out by routine techniques known in the art.

The method in its entirety may be performed multiple times (e.g. 2, 3 or more times) after an appropriate interval or parts of the method may be repeated if necessary.

In one embodiment the method comprises the following steps:
(i) Identifying an antigen for the condition;
(ii) Identifying a tolerogenic peptide for the antigen; and
(iii) Administering the tolerogenic peptide to the subject according to the present invention.

In a further embodiment the method comprises identifying a subject with a condition associated with aberrant or pathological immune tolerance or identifying a subject who is likely to develop a condition associated with aberrant or pathological immune tolerance.

The peptide may be in the form of a composition, comprising and one or more pharmaceutically acceptable diluents, carriers or excipients. These compositions may be formulated in any convenient manner according to techniques and procedures known in the pharmaceutical art, e.g. using one or more pharmaceutically acceptable diluents, carriers or excipients.

The composition may by prepared as an injectable, either as liquid solution or suspension; solid form suitable for solution in, or suspension in, liquid prior to injection may also be prepared. The preparation may also be emulsified, or the peptides encapsulated in liposomes. The peptide may alternatively be encapsulated in a carrier or bound to the surface of a carrier, for example a nanoparticle. The active ingredients may be mixed with excipients which are pharmaceutically acceptable and compatible with the active ingredient.

"Pharmaceutically acceptable" as referred to herein refers to ingredients that are compatible with other ingredients of the compositions as well as physiologically acceptable to the recipient.

Suitable excipients are, for example, water, saline (for example, phosphate-buffered saline), dextrose, glycerol, ethanol, or the like and combinations thereof.

In addition, if desired, the composition may contain minor amounts of auxiliary substances such as wetting or emulsifying agents and/or pH buffering agents. Buffering salts include phosphate, citrate, acetate. Hydrochloric acid and/or sodium hydroxide may be used for pH adjustment. For stabilisation, disaccharides may be used such as sucrose or trehalose.

The nature of the composition and carriers or excipient materials etc. may be selected in routine manner according to choice and the desired route of administration, purpose of treatment etc.

The peptide may be administered in soluble form in the absence of an adjuvant.

The present inventors predict that, despite "bystander suppression" it may be necessary to target a number of different T cell clones in order to induce tolerance effectively. Hence a plurality of peptides, i.e. more than one peptide, may be administered to an individual in order to prevent or treat a disease.

The pharmaceutical composition may, for example comprise between 1 and 50 peptides, preferably between 2 and 15 peptides. The peptides may be derivable from the same or different target antigen(s). Preferably the peptides are either all able to bind to MHC class I, or all able to bind MHC class II, without further processing, i.e. they are apitopes. In a preferred embodiment all the apitopes in the pharmaceutical composition are either able to bind to MHC class I or class II without further processing.

The pharmaceutical composition may be in the form of a kit for use according to the invention, in which some or each of the peptides is provided separately for simultaneous, separate or sequential administration.

Alternatively (or in addition) if the pharmaceutical composition (or any part thereof) is to be administered in multiple doses, each dose may be packaged separately.

The pharmaceutical composition may comprise a therapeutically or prophylactically effective amount of the or each peptide and optionally a pharmaceutically acceptable carrier, diluent or excipient.

Also, in the pharmaceutical compositions of the present invention, the or each peptide may be admixed with any suitable binder(s), lubricant(s), suspending agent(s), coating agent(s), or solubilising agent(s).

### EXAMPLES

The following examples serve to illustrate the present invention, but should not be construed as a limitation thereof. The invention particularly relates to the specific embodiments described in these examples

### EXAMPLE 1

An open label ascending dose evaluation of the safety, immunology and effect on an imaging based biomarker of efficacy (gadolinium enhanced MRI scanning) of ATX MS 1467 was carried out in 40 patients who are HLA DRB1*15 positive with relapsing multiple sclerosis.

### Methodology:

Phase I, open label, ascending dose study. Eight week titration period (25, 50, 100, 400 and 800 µg administered at intervals of 14±3 days); 6 week full dose treatment period (800 µg administered on 5 occasions at intervals of 14±3 days); 28 week follow up period (no treatment with study medication). Patients in Cohort 1 were treated with ATX-MS-1467 intradermally (i.d.); patients in Cohort 2 were treated with ATX-MS-1467 subcutaneously (s.c.). The combined safety data for Cohort 1 was subject to an interim safety review by the Data Monitoring Committee (DMC) after the last dose of 800ug was administered to the first 10 and 20 patients, respectively.

| **Number of Patients (Planned and Analyzed):** | | | | | | | |
|---|---|---|---|---|---|---|---|
| | | **Cohort 1 (i.d.)** | | **Cohort 2 (s.c.)** | | **Total** | |
| | | n | % | n | % | n | % |
| Overall - Study Population Planned* | | 20 | | 20 | | 40 | |
| Study population | | 21 | | 22 | | 43 | |
| | ITT | 21 | (100%) | 22 | (100%) | 43 | (100 |
| | PP | 16 | (76.2%) | 19 | (86.4%) | 35 | (81.4 |
| | MRI | 17 | (81.0%) | 20 | (90.9%) | 37 | (86.0 |
| Completed Study | | 19 | (90.5%) | 20 | (90.9%) | 39 | (90.7 |
| Did Not Complete Study | | 2 | (9.5%) | 2 | (9.1%) | 4 | (9.3 |
| Reason for Withdrawal from Study | | | | | | | |
| | Patient requests early discontinuation | 1 | (4.8%) | 2 | (9.1%) | 3 | (7.0 |
| | Patient lost to follow-up | 1 | (4.8%) | 0 | | 1 | (2.3 |
| Treatment Phase - ITT Population** | | | | | | | |
| Completed treatment | | 20 | (95.2%) | 22 | (100%) | 42 | (97.7 |
| Did Not Complete Treatment | | 1 | (4.8%) | 0 | | 1 | (2.3 |
| Reason for Withdrawal from Treatment | | | | | | | |
| | Adverse Event | 1 | (4.8%) | 0 | | 1 | (2.3 |

| | | | | | | | |
|---|---|---|---|---|---|---|---|
| *Percentages are based on the number of patients in the Study population. **Percentages are based on the number of patients in the ITT population. i.d. = intradermal, ITT = intention-to-treat, MRI = magnetic resonance imaging, PP = per protocol, s.c. = subcutaneous | | | | | | | |

### Diagnosis and Main Criteria for Inclusion:

Diagnosis: human lymphocyte antigen (HLA)-DRB1*15 positive with RMS as defined by the McDonald criteria and as assessed by a neurologist
Main Criteria for Inclusion:
   - High baseline levels of T-cell proliferation in response to MBP, defined as > 1000 counts per minute (cpm) with a > 3 stimulation index compared to background.
   - Disease duration ≤ 10 years (from the first clinical event).
   - At least one documented relapse in the previous 12 months or 2 relapses within the previous 24 months prior to screening.
   - Expanded disability status scale (EDSS) score ≤ 5.5.
   - 18 - 55 years inclusive at the time of informed consent.
   - Contraceptive use, for males and females, as defined in the Protocol.

Willing and able to give written informed consent.

### Test Product, Dose and Mode of Administration:

| | |
|---|---|
| **ATX-MS-1467A:** | 4 mg/mL mixture made of equal parts of the peptides ATX-MS-01, ATX-MS-04, ATX-MS-06 and ATX-MS-07 in phosphate buffered saline (PBS) |
| **ATX-MS-1467B:** | 0.5 mg/mL mixture made of equal parts of the peptides ATX-MS-01, ATX-MS-04, ATX-MS-06 and ATX-MS-07 in PBS |

| **Doses Administered During the Study** | | | | | |
|---|---|---|---|---|---|
| **Dose number** | **Total dose (µg)** | **Dose of each peptide (µg)** | **Injection volume (µL)** | **ATX-MS-1467A or ATX-MS-1467B** | **Number of injections** |
| 1 | 25 | 6.25 | 50 | B | 1 |
| 2 | 50 | 12.5 | 100 | B | 1 |
| 3 | 100 | 25 | 200 | B | 2 |
| 4 | 400 | 100 | 100 | A | 1 |
| 5 | 800 | 200 | 200 | A | 2 |
| 6 | 800 | 200 | 200 | A | 2 |
| 7 | 800 | 200 | 200 | A | 2 |
| 8 | 800 | 200 | 200 | A | 2 |
| 9 | 800 | 200 | 200 | A | 2 |

### Mode of Administration:

Cohort 1: i.d.
Cohort 2: s.c.

### Duration of Treatment:

Patients were to receive 1 or 2 injections of ATX-MS-1467 at intervals of 14±3 days for 16 weeks.

### Criteria for Evaluation:

### Efficacy

### Magnetic Resonance Imaging

- MRI New Gd-enhancing lesions, T2 hyperintense lesions, T1 hypointense lesions. Week -4, Study Day 1; in the week prior to or at the Week 4 and Week 8 visit; in the week prior to visits at Weeks 12, 16, 20, 28, 36 and 48, every follow-up visit; Early Termination Visit. MRls were assessed as part of safety and efficacy.

### Safety

| | |
|---|---|
| • Adverse events (AEs): | severity, relationship to ATX-MS-1467, outcome, action taken, onset, resolution date, seriousness. Recorded throughout the study. |
| • Injection site reactions: | at the time of every injection. |
| • Vital signs: | heart rate, systolic and diastolic blood pressure, body temperature. Screening, Week -4, at each injection visit. |
| • Physical examination: | Screening, Week -4, at each injection visit; Early Termination Visit. |
| • 12 lead ECG: | Screening and Week -4. |
| • Neurological examination | Kurtzke Functional System (KFS) and (Kurtzke) Expanded Disability Status Scale (EDSS). Screening, Week -4, Study Day 1, Week 12, every follow-up visit; Early Termination Visit. |
| • Hematology: | red blood cell count, hemoglobin, hematocrit, mean corpuscular hemoglobin [MCH], mean cell hemoglobin concentration [MCHC], total and differential white cell count, platelet count. At every visit. |
| • Clinical chemistry: | urea, sodium, potassium, chloride, bicarbonate, creatinine, total bilirubin, ALT, AST, alkaline phosphatase [ALP], serum proteins, uric acid, calcium, phosphate. At every visit. |
| • Urinalysis: | at every visit. |
| • Thyroid function test: | T3, T4 and TSH. Screening, Week -4 if abnormal results obtained at Screening, Week 48; Early Termination Visit. |
| • Anti-peptide antibody test: | Screening. At Week 16, Week 20 on patients that were HLA-DRB1*15 positive at Screening. |
| • MS attacks: | at every visit from Week -4. |
| • Urine pregnancy test: | Screening, Week -4, at each injection visit, first follow-up visit (Week 20); Early Termination Visit. |

### Statistical Methods:

No formal sample size calculations and no formal interim analyses were performed (no confirmatory statistical testing). P-values were presented for descriptively purposes only. Trial investigations were to be exploratory and conclusions were based on the complete set of patient evidence.

Categorical variables were presented using the number of non-missing observations (n) or the number of patients in the population (N) as applicable and percentages (%). Percentages were rounded to one decimal place. Two-sided 95% Confidence Intervals (CI) were provided when relevant. Continuous variables were described by number of non-missing observations (n), arithmetic mean (Mean), standard deviation (SD), minimum (Minimum), median (Median), Q1-Q3, and maximum (Maximum).

Efficacy analyses were based on the PP population (i.e. the evaluable patients). The analysis of the MRI evaluation of brain lesions (proof of principle) was based on the MRI population. All safety analyses were based on the ITT population. Unless otherwise stated, the baseline value for each patient presented in summary tables was defined to be the latest non-missing assessment value for a patient, for that particular parameter that was prior to the initiation of IMP on Study Day 1, including unscheduled or repeat assessments.

The ITT population was defined as all patients who received at least one administration of IMP any time during the study, irrespective of compliance with eligibility and other protocol criteria. The Safety population was denoted as the ITT population for summarization of safety endpoints. The PP population (evaluable patients) was defined as all ITT patients who complied with the protocol (i.e., no major protocol violations, satisfied inclusion/exclusion criteria, received the full treatment regimen, took no steroids or other prohibited medication during the treatment phase) up to and including the Week 20 visit. The MRI population was defined as the PP population plus those patients who had major violations for PD assessments. The MRI population was used to analyze brain lesions (proof of principle).

### Summary of results

### Efficacy, MRI

In the MRI population of Cohort 1, there was an overall decrease in the mean number of new or persisting Gd-enhancing lesions from 3.4 (SD: 8.23) at baseline to 2.5 (SD: 4.91) at Week 48/ET; the decrease from baseline to Week 16 (0.9 lesions; SD: 1.67) was statistically significant (p = 0.030). These trends were also seen in the ITT population, with a statistically significant decrease in the mean number of new or persisting Gd-enhancing lesions from Study Day 1 (5.0, SD: 9.76) to Week 16 (1.2, SD: 1.81; p < 0.001) and an increase from Week 16 through Week 48/ET to 3.2 (SD: 5.33). There was also an overall decrease in the total volume of Gd-enhancing lesions in Cohort 1 of -104.8 mm³, from 304.8 mm³ at baseline to 200.1 mm³ at Week 48.

In Cohort 2, the mean number of new or persisting Gd-enhancing lesions was comparable from baseline (2.3; SD: 5.47) to Week 48/ET (2.1; SD: 3.63). The increase in the mean number of new or persisting Gd-enhancing lesions from baseline (2.3 lesions) to Week 16 (3.5 lesions; SD: 7.09) was statistically significant (p = 0.012) despite the small change in the mean number of lesions. The trends in the ITT population of Cohort 2 also reflected those seen in the MRI population of Cohort 2. The increase in the mean number of new or persisting Gd-enhancing lesions was statistically significant (p = 0.010) though the actual number of lesions seen at both time points was comparable (Study Day 1: 2.1 lesions; SD: 5.25; Week 16: 3.2 lesions; SD: 6.80;); the mean number of new or persisting lesions decreased from Week 16 to Week 48/ET (2.0 lesions, SD: 3.48). The mean total volume of Gd-enhancing lesions in Cohort 2 was comparable (overall change of +13.3 mm³) from baseline (188.6 mm³) to Week 48 (201.0 mm³).

The mean number of new or newly enlarging T2 hyperintense lesions in Cohort 1 (MRI population) was comparable from Week 8 (5.1 lesions; SD: 8.35) to Week 48 (5.2 lesions; SD: 9.44). In Cohort 2, the mean number of lesions increased slightly from Week 8 (3.5; SD: 7.07) to Week 48 (4.1; SD: 6.64).

The mean cumulative number of new T1 hypointense lesions (assessed at Week 48 only, compared to baseline) was 7.2 (SD: 12.79) in Cohort 1 and 7.8 (SD: 9.21) in Cohort 2; the total number of new T1 hypointense lesions at Week 48 was 7.5 (SD: 10.87).

In the MRI population of Cohort 1, the proportion of patients free of GD-enhancing lesions increased from 52.9% (9/17) at Study Day 1 to 70.6% (12/17) at Week 48. In Cohort 2, the number of patients free of GD enhancing lesions was comparable at Study Day 1 (65.0%, 13/20) and Week 48 (59.5%, 22/37).

In Cohort 1, the proportion of patients free of T2 lesions was comparable at Week 28 (52.9%, 9/17) and Week 48 (56.3%, 9/16). In Cohort 2, there was a slight decrease in the proportion of patients T2 lesion free from Week 28 (35.0%, 7/20) to Week 48 (27.8%, 5/18). Assessments of patients which were T1 lesion-free were done at/up to Week 48 only. At this time point, 38.2% (13/34) of all patients in the MRI population were lesion free, including 50.0% (8/16) of patients in Cohort 1 and 27.8% (5/18) of patients in Cohort 2.

### Safety:

### Treatment Emergent Adverse Events

### Global Overview of Treatment Emergent Adverse Events - ITT Population

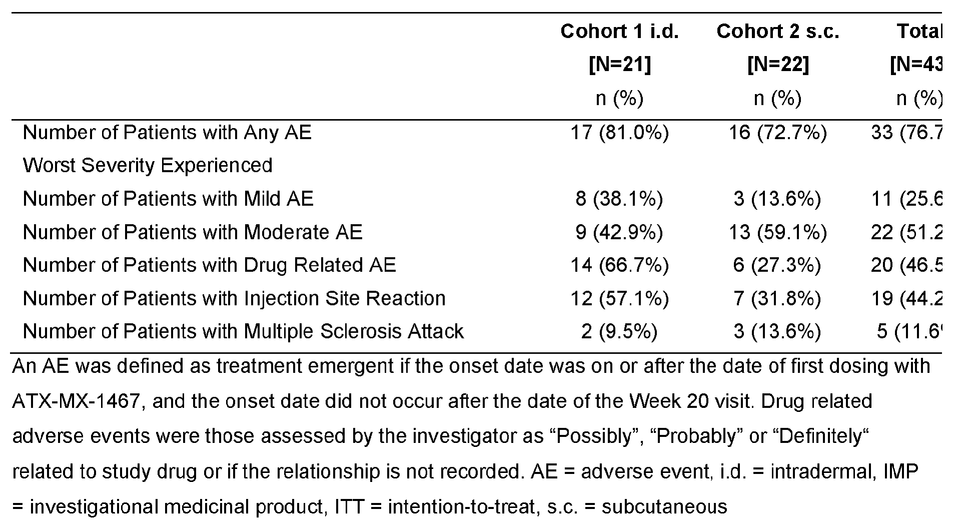

There were no severe TEAEs; no AEs or SAEs associated with death; no treatment related SAEs; and no AEs that resulted in dose suspension or withdrawal from the study. There were no antibody responses to any peptides to ATX-MS- 1467 in either Cohort. Only 1 patient (2.3%; Cohort 1) had an AE that led to drug interruption (pruritic rash 1 week after receiving 800 µg of study medication at Week 14). There were no clinically relevant changes seen in laboratory values, vital signs or Kurtzke functions (mean values or mean changes from baseline) seen during the study. The number of MS relapses was not unexpected for this patient population.

Only 1 patient experienced a treatment emergent SAE (Cohort 1; MS relapse; 800 µg at Week 17); the event was assessed as not related to ATX-MS-1467.

### Treatment Emergent Adverse Events by Dose - ITT Population

| | Dose (µg) | | | | |
|---|---|---|---|---|---|
| | 25 Week 0 n (%) | 50 Week 2 n (%) | 100 Week 4 n (%) | 400 Week 6 n (%) | 800 µg Weeks 8 n (%) |
| Cohort 1, N=21 | 6 (28.6%) | 10 (47.6%) | 12(57.1%) | 7 (33.3%) | 17 (81.0 |
| Cohort 2, N=22 | 4 (18.2%) | 7 (31.8%) | 5 (22.7%) | 4 (18.2%) | 12 (54.5 |

In general, the greater number of patients reporting a TEAE in Cohort 1 was due to the higher number of injection site reactions. While the number of patients reporting an AE tended to be highest at the combined 800 µg dose level, it is important to note that the combined doses of 800 µg includes data from 5 time points (Weeks 8, 10, 12, 14, and 16).

In Cohort 1, the most frequently reported TEAEs at all dose levels were seen in the General disorders and administration site conditions SOC, with the most frequently reported TEAE by preferred term at all dose levels being injection site erythema. Nasopharyngitis was experienced by 3 patients (14.3%) at the 800 µg dose level only. Multiple sclerosis relapse, experienced by 2 patients (9.5%), was also seen only at the 800 µg dose level (Week 14 and Week 16). There were no other TEAEs, by preferred term, reported by more than 1 patient in Cohort 1.

In Cohort 2, the most frequently reported TEAEs overall were seen in the Nervous system disorders SOC, with the most frequently reported TEAE by preferred term being headache. Multiple sclerosis relapse was experienced by 1 patient (4.5%) at the 400 µg dose level and 3 patients (13.6%) in the combined 800 µg dose level (2 patients at Week 8, 1 patient at Week 16). There were no other TEAEs, by preferred term, reported by more than 1 patient in Cohort 2.

Two patients in Cohort 1 and 3 patients in Cohort 2 experienced treatment emergent MS attacks. In Cohort 1, both patients experienced the MS attack after treatment at 800 µg. In Cohort 2, 1 patient experienced an MS attack after treatment at 400 µg and 3 patients after treatment at 800 µg. None of the events were assessed as related to treatment, none of the events resulted in any action with respect to the administration of the study medication and all patients recovered; only 1 event (Cohort 1) was assessed as serious. All attacks were assessed as being of mild or moderate severity.

### Treatment-Related Adverse Events

The higher number of treatment-related AEs in Cohort 1 was driven by the higher number of injection site reactions experienced in this cohort. In Cohort 1, the most frequently reported drug related TEAE at all dose levels was injection site erythema (100 µg: n=8, 38.1%; combined 800 µg: n=9, 42.9%). One patient (4.8%) reported treatment-related pyrexia (50 µg) and 1 patient reported treatment-related influenza-like illness (100 µg).

I n Cohort 2, the most frequently reported drug related event reported was also injection site erythema (combined 800 µg: n=3, 13.6%). There were no other drug related events reported by more than 1 patient in Cohort 2 at any dose levels. There were no reports of treatment-related pyrexia or influenza-like illness in Cohort 2.

All treatment emergent injection site reactions were assessed as mild, in both cohorts. All TEAEs of influenza-like illness and pyrexia were of moderate severity. There were no reports of drug-related MS relapse in either cohort.

### Treatment Emergent Injection Site Reactions, Cohort 1 - ITT Population

| | Dose (µg) | | | | |
|---|---|---|---|---|---|
| | 25 Week 0 n (%) | 50 Week 2 n (%) | 100 Week 4 n (%) | 400 Week 6 n (%) | 800 µg Weeks 8 n (%) |
| Erythema | 4 (19.0%) | 7 (33.3%) | 9 (42.9%) | 5 (23.8%) | 9 (42.9 |
| Induration | 2 (9.5%) | 1 (4.8%) | 1 (4.8%) | 0 | 4 (19.0 |
| Pain | 1 (4.8%) | 1 (4.8%) | 2 (9.5%) | 1 (4.8%) | 3 (14.3 |

### Treatment Emergent Injection Site Reactions, Cohort 2 - ITT Population

| | Dose (µg) | | | | |
|---|---|---|---|---|---|
| | 25 Week 0 n (%) | 50 Week 2 n (%) | 100 Week 4 n (%) | 400 Week 6 n (%) | 800 µg Weeks 8 n (%) |
| Erythema | 0 | 0 | 1 (4.5%) | 0 | 3 (13.6% |
| Induration | 0 | 1 (4.5%) | 0 | 0 | 1 (4.5% |
| Pain | 1 (4.5%) | 0 | 1 (4.5%) | 0 | 0 |
| Tenderness | 0 | 0 | 0 | 1 (4.5%) | 0 |

### Global Overview of Non-Treatment Emergent Adverse Events (Post Treatment) - ITT Population

| | **Cohort 1 i.d. [N=21]** n (%) | **Cohort 2 s.c. [N=22]** n (%) | **Total [N=43]** N (%) |
|---|---|---|---|
| Number of Patients with Any AE | 9 (42.9%) | 8 (36.4%) | 17 (39.5 |
| Worst Severity Experienced | | | |
| Number of Patients with Mild AE | 1 (4.8%) | 1 (4.5%) | 2 (4.7% |
| Number of Patients with Moderate AE | 8 (38.1%) | 7 (31.8%) | 15 (34.9% |
| Number of Patients with SAE | 2 (9.5%) | 1 (4.5%) | 3 (7.0% |
| Number of Patients with Drug Related AE | 2 (9.5%) | 1 (4.5%) | 3 (7.0% |
| Number of Patients with Multiple Sclerosis Attack | 4 (19.0%) | 4 (18.2%) | 8 (18.6% |

An AE was defined as non-treatment emergent starting post-treatment if the onset date was after the date of the Week 20 visit.

Drug related adverse events are those assessed by the investigator as "Possibly", "Probably" or "Definitely" related to study drug or if the relationship is not recorded.
AE = adverse event, i.d. = intradermal, ITT = intention-to-treat, s.c. = subcutaneous

Post treatment, there were no severe AEs; no AEs or SAEs associated with death; no AEs that led to withdrawal from the study; no injection site reactions; and no treatment-related SAEs.

Four patients in Cohort 1 and 4 patients in Cohort 2 experienced a post-treatment relapse of MS; 2 patients in Cohort 1 experienced multiple post-treatment relapses. Two post-treatment relapses experienced by a Cohort 1 patient were assessed as possibly related to treatment, but not serious. Two patients in Cohort 1 experienced a post-treatment relapse assessed as serious, but neither event was assessed as related to treatment. None of the post-treatment relapses seen in Cohort 2 were assessed as related to study treatment. One post-treatment relapse reported by a patient in Cohort 2 was assessed as serious. All attacks were assessed as moderate.

### Example 2

An Open-label, One-arm, Proof of Concept Trial to Evaluate the Safety of ATX-MS-1467 (MSC2358825A) and its Effect on Immune Tolerance in Subjects with Relapsing Multiple Sclerosis was carried out.

### Methodology:

This was a multi-centre, open-label, Phase Ila study in subjects with relapsing MS. The study consisted of 5 periods; subjects remained in the study for 48 weeks.

Screening Period (4 weeks): Prior to entering the Baseline Control Period, subjects were screened to establish their initial eligibility. Subjects were required to have completed any prior treatment with corticosteroids at least 30 days prior to their first MRI scan at Visit 2. Subjects taking any other non-permitted MS therapy at Visit 1 discontinued all such medications as soon as possible after it had been confirmed they had the human leukocyte antigen (HLA) DRB1*15 genotype and were eligible for the study based on their Visit 2 MRI scan.

Baseline Control Period (8 weeks/3 visits): Subjects who were HLA-positive underwent 3 brain MRI scans (with a minimum interval of 28 days between successive scans) to determine their eligibility based on degree of MRI activity. Following confirmation of subject eligibility with respect to MRI criteria, subjects could have had an optional lumbar puncture for the collection of cerebrospinal fluid (CSF). During the Baseline Control Period, subjects did not receive treatment for MS.

Titration Period (4 weeks/3 visits): Following completion of the Baseline Control Period, eligible subjects entered the Titration Period during which biweekly ATX-MS-1467 ID was titrated from the starting dose (50 µg) to the maximum dose (800 µg) according to the following schedule:
Day 1: ATX-MS-1467 50 µg ID
Day 15: ATX-MS-1467 200 µg ID
Day 29: ATX-MS-1467 800 µg ID.

Treatment Period (16 weeks/8 visits): During the Treatment Period, subjects received biweekly dosing with ATX-MS-1467 800 µg ID for 16 weeks and attended study visits for dosing and safety evaluation at 2-week intervals; additional clinical evaluations, including MRI scans, were performed on 3 occasions during the Treatment Period.

Follow-up Period (16 weeks/4 visits): Following completion of the Treatment Period, subjects entered the 16-week Follow-up Period. Subjects remained off study treatment during this period and were evaluated for the maintenance of any therapeutic effect. The MRI scans were performed on 3 occasions during the Follow-up Period.

Subjects who prematurely withdrew from treatment with ATX-MS-1467, i.e., who withdrew during the Titration or Treatment Periods, entered an 8-week Safety Follow-up Period (2 visits).

### Number of Subjects:

At least 15 evaluable subjects were planned for study participation.

A total of 93 subjects were screened for study participation with 37 (39.8%) subjects enrolled in the study to begin the Baseline Control Period. There were 19 subjects who entered and completed the Titration Period and entered the Treatment Period. All 19 subjects treated were included in the Intention-to-Treat (ITT), Modified ITT (mITT), and Safety Analysis Sets.

### Diagnosis and Main Criteria for Inclusion:

Male and female outpatients, 18 to 65 years of age, inclusive, who had relapsing-remitting MS (RRMS) or secondary progressive MS (SPMS) according to the McDonald diagnostic criteria (2010), clinical evidence of recent MS activity, at least 1 contrast-enhanced lesion (CEL) on MRI at Visit 2, and the presence of at least 1 new CEL from Visit 2 to Visit 4 were eligible for study participation. Subjects had to have an Expanded Disability Status Scale (EDSS) score between 0 and 5.5, be HLA DRB1*15-positive, and be neurologically stable prior to the start of study treatment. Subjects were not eligible for this study if they had primary progressive MS, renal conditions that precluded the administration of gadolinium (Gd), lymphocyte count < 500/µL or neutrophil count < 1500/µL at pretreatment visits, or other underlying medical conditions that precluded participation in the study.

Previous treatments with beta-interferon, plasma exchange, or intravenous gamma globulin within the 8 weeks prior to Study Day 1; steroids or adrenocorticotropic hormone within the 30 days prior to the Visit 2 MRI scan; or glatiramer acetate, cytotoxic agents, fingolimod, laquinimod, teriflunomide, total lymphoid irradiation, stem cell or bone marrow transplantation, monoclonal antibody therapy, dimethyl fumarate, dirucotide, any disease related T-cell vaccine or peptide-tolerizing agent for the treatment of MS were not allowed.

### Test Product(s): Dose and Mode of Administration, Batch Number(s):

The investigational medicinal product was ATX-MS-1467 and was administered ID biweekly for a total of 20 weeks, from a starting dose of 50 µg and titrated over 4 weeks to the final dose of 800 µg. Individual batch numbers are available upon request.

### Duration of Treatment:

From Visit 1 to Visit 19, the maximum duration of study participation was 48 weeks. The duration of study treatment was 20 weeks.

### Reference Therapy(ies), Dose and Mode of Administration, Batch Number(s):

Not applicable.

### Criteria for Evaluation:

### Efficacy:

The primary endpoint was the change in the average number of T1 CEL at the last 3 on-treatment scans (Weeks 12, 16, and 20) compared to the average number of T1 CEL at the 3 baseline scans (Visits, 2, 3, and 4).

Secondary endpoints included the following:
- MRI
- Total number of T1 CEL at each scheduled post-baseline MRI visit
- Change from baseline (average of the 3 baseline scans, Visits 2, 3, and 4) in total number of T1 CEL at each scheduled post-baseline MRI visit
- Change from baseline (average of the 3 baseline scans, Visits 2, 3, and 4) in total volume of T1 CEL at each scheduled post-baseline MRI visit
- Total number of new or newly enlarging T2 lesions at each scheduled post-baseline MRI visit
- Change from Visit 4 in total number of T1 CEL at each scheduled post-baseline MRI visit
- Change from Visit 4 in total volume of T1 CEL at each scheduled post-baseline MRI visit.
- Clinical
- Mean annualized relapse rate (ARR) at Week 20
- Time to first relapse
- Change from baseline in total EDSS score at Week 20
- Change from baseline in total Multiple Sclerosis Functional Composite (MSFC) score at Week 20.

The following exploratory endpoints were also considered:
- Maintenance of the effects of ATX-MS-1467 on MRI parameters following discontinuation of treatment at Weeks 28 and 36 in the subgroup of responders
- Modulation of immunological markers (e.g., cytokines, antibodies) in peripheral
- blood/peripheral blood mononuclear cells (PBMC) and in CSF over time
- Modifications of disease markers (e.g., OCB, immunoglobulin G [lgG] index, demyelination)
- in peripheral blood and in CSF over time
- Serum anti-peptide antibody levels at Weeks 11, 20, and 24
- Changes in MBP-induced PBMC proliferation over time:
- Changes from baseline in MBP-induced PBMC proliferation at Week 20
- Changes from Week 20 in MBP-induced PBMC proliferation at Week 36/End-of-Study.

### Safety:

Safety endpoints included the following:
- Nature, frequency, and severity of treatment-emergent adverse events (TEAEs)
- Frequency and severity of injection site reactions
- Vital sign measurements, physical examination findings, clinical laboratory variables, electrocardiograms, and the frequency and timing of premature termination from the study.

### Statistical Methods:

The sample size for this study was based on 3 assumptions: (1) A treatment effect of 70% reduction in the number of CEL as compared to the average number of CEL at the 3 baseline scans; (2) the mean number of CEL at baseline was 5 with a standard deviation (SD) of 6; and (3) the mean number of CEL during the post-treatment period (Weeks 24 to 36) was 1.5 with a SD of 1.8. Using a 2-sided 5% level, > 80% and > 90% of the simulated studies showed a statistically significant result with sample sizes of 12 and 14 subjects, respectively. Thus, a sample size of 15 subjects was selected.

Analysis of the primary and secondary endpoints was based on the mITT Population, except for evaluation of the maintenance of response which was performed using the Responders Population and safety analyses which were based on the Safety Population. Responders were defined as those subjects in whom there was a reduction from baseline (average of the 3 baseline scans at Visits 2, 3, and 4) of ≥ 60% in the number of T1 Gd enhancing lesions at Week 20 (average of the last 3 on-treatment scans at Weeks 12, 16, and 20).

The primary endpoint was analyzed using a nonparametric Wilcoxon Signed Rank test for a test of shift in location due to the treatment effect based on an exact distribution of the signed rank statistic where the distribution is a convolution of scaled binomial distributions. A supportive analysis was performed to estimate mean percentage reduction in new T1 Gd-enhancing lesions during the treatment period compared to baseline control period using generalized estimating equations (GEE) linear regression model with negative binomial and Poisson link functions.

The same nonparametric procedure used for the primary efficacy endpoint was carried out based on new T1 Gd-enhancing lesions. Descriptive statistics were displayed for secondary endpoints at each applicable visit for the mITT Analysis Set.

Continuous variables were summarized descriptively using the number of observations, means, SD, 95% confidence interval (CI), median, minimum, and maximum. Categorical variables were summarized using frequency counts and percentages. Time to event variables were presented as Kaplan Meier plots, median survival, and 95% Cl.

### Summary and Conclusions:

### Subject Disposition:

There were 93 subjects screened for study participation; 37 (39.8%) subjects were enrolled. Nineteen (51.4%) subjects entered and completed the Titration Period and 18 (48.6%) subjects completed the Treatment Period. One subject (2.7%) discontinued investigational medicinal product (IMP) due to an adverse event (AE) of diarrhea. A second subject (2.7%) withdrew consent after completing the Treatment Period. All 19 eligible subjects were included in the ITT, mITT, and Safety Analysis Sets. Seven subjects who demonstrated a reduction from baseline in the number of T1 Gd enhancing lesions of ≥ 60% at Week 20 were included in the Responders Analysis Set.

### Demographics and Baseline Characteristics:

The mean age of subjects in the study was 27.1 years (range 19 to 38 years) with the majority of subjects < 30 years of age (73.7%). Most subjects were female (78.9%) and all subjects were white. All 19 subjects had a diagnosis of RRMS with the majority of subjects (89.5%) reporting 1 to 2 relapses in the 24 months prior to Visit 2. The median EDSS score at baseline was 2.00 (range 1.5 to 3.5). The median MSFC score at baseline based on the National Multiple Sclerosis Society(NMSS) reference population was 0.470 (range -0.95 to 1.21). The mean number and volume of T1 Gd-enhancing lesions were 7.4 (range 1 to 31) and 0.838 mL (range 0.05 to 3.65 mL), respectively.

### Efficacy Results:

There was a statistically significant decrease in the average number of T1 Gd-enhancing lesions on treatment (Weeks 12, 16, and 20) compared with baseline based on a nonparametric analysis using the mITT analysis set (p = 0.0143). The Hodges-Lehmann estimate of location shift (95% Cl) was -1.3 (-6.3, 0.0). Similarly, there was a statistically significant decrease in average number of new T1 Gd-enhancing lesions (p = 0.0106). The Hodges-Lehmann estimate of location shift (95% Cl) was -1.3 (-5.7, 0.0). The results of supportive analysis of new T1 Gd-enhancing lesions are consistent with the primary analysis.

Numerical decreases from baseline in mean T1 Gd-enhancing lesion count and volume were noted at all postdose assessments. The mean number of lesions at baseline was 7.4 (range 1 to 31) and mean change from-baseline in lesion count ranged from -4.6 to -1.6. The mean lesion volume at baseline was 0.838 mL (range 0.05 to 3.65 mL) and the mean change from baseline in lesion volume ranged from -0.579 to -0.225 mL. Similarly, numerical decreases in mean T1 Gd-enhancing lesions and volumes from Week 0 to each postdose assessment were noted. The mean change from Week 0 in lesion count ranged from -3.5 to -0.9 and the mean change from Week 0 in lesion volume ranged from -0.473 to -0.157 mL. The median number of new T1 Gd-enhancing lesions was similar from Week 12 (1.5) through the end of study (ranging from 0.0 at Week 28 to 2.0 at End of Study). The median number of new/enlarging T2 lesions decreased from 8.0 (range 0 to 89) at Week 12 to 1.0 at Week 16 (range 0 to 20), and the median count ranged from 1.0 to 3.0 at subsequent visits.

During study treatment, a single relapse occurred in 3 (15.8%) subjects; no relapse during treatment was reported for the remaining 16 subjects. The estimated mean ARR was 2.60. For these 3 subjects, the onset of relapse occurred on Days 50, 59, and 89 and the Kaplan-Meier estimates of the probability of not experiencing relapse decreased from 1.00 at Week 4 to 0.84 at Week 20.

The change from baseline to the End of Treatment visit in EDSS score was not statistically significant. Similarly, the changes from baseline to the End of Treatment visit in MSFC scores were not statistically significant when using either baseline values from enrolled subjects or values from the NMSS Task Force Database as reference.

The average percentage of treatment effect maintained was ≥ 85% for 4 (57.1%) of the 7 subjects considered responders.

### Safety Results:

Overall, 78.9% of subjects reported at least 1 TEAE during study conduct; TEAEs in 57.9% of subjects were assessed as related to IMP. The most frequently-reported TEAEs were injection site erythema (26.3%), headache (21.1%), and nasopharyngitis (15.8%).

There were no deaths, serious TEAEs, or TEAEs of severe intensity reported. One subject discontinued IMP due to a TEAE of diarrhea, which was prolonged in duration and assessed as moderate in intensity and related to IMP.

The onset of TEAEs for most subjects was < 26 days from the start of IMP.

All injection site reactions (36.8% of subjects) during the study were of mild intensity and the most frequently-reported symptoms were erythema, pruritus, and induration.

Three subjects had a ≥ 7% decrease in weight during the study; otherwise, there were no clinically relevant changes in clinical laboratory, vital sign, or electrocardiogram parameters.

### Conclusions:

The median decrease in T1 Gd-enhancing lesions during the treatment period compared to the baseline control period was statistically significant (p = 0.0143) based on a nonparametric Wilcoxon signed rank test. The Hodges-Lehmann estimate of location shift (95% Cl) was -1.3 (-6.3, 0.0).

Supportive analyses using negative binomial and Poisson GEE models yielded consistent findings. Compared to the baseline control period, the mean percentage reduction in new T1 Gd-enhancing lesions during the treatment period was statistically significant (p value = 0.0109). The GEE-estimated mean percentage reduction (95% Cl) using the negative binomial model was 38.4% (10.6%, 57.6%).

Numerical decreases from baseline and from Week 0 in mean T1 Gd-enhancing lesion count and volume were noted at all postdose assessments.

The median number of new/enlarging T2 lesions decreased from 8.0 at Week 12 to 1.0 at Week 16, and ranged from 1.0 to 3.0 at subsequent visits.

Few subjects (15.8%) experienced relapse during the study. The Kaplan-Meier estimated probability of not experiencing relapse by Week 20 is 0.84.

No statistically significant changes from baseline to the End of Treatment visit in EDSS or MSFC scores were noted.

For the 7 subjects considered responders, the average percentage of treatment effect maintained was ≥ 50%; however, 3 of these subjects had very low baseline lesion counts and therefore achieved 100% reduction.

No safety or tolerability concerns were identified following treatment with ATX-MS-1467 800 µg administered ID every 2 weeks.

### Example 3

The results of the two trials in Examples 1 and 2 were compared. As shown in Figures 1 and 2, there was a significant decrease in new lesions confirming efficacy in both trials. However, Figure 1 shows that the dose titration schedule is important in maximising efficacy. The dose titration used in the first trial resulted in a 78% reduction in the number of new lesions, whereas Figure 2 shows that the shorter dose titration schedule used in the second trial did not lead to this level of reduction. On the other hand, Figure 2 shows that repeated treatment at the maximum dose (800µg) prolonged the efficacy. As such, for maximum efficacy the longer dose escalation from the first trial should be used in combination with the extended treatment at the maximum dose as used in the second trial.

### Example 4

Safety and proof of principle study of ATX-GD-59 in male and female subjects with Graves' disease not currently treated with anti-thyroid therapy: An Open label study, with an upward titration over five dose levels administered by Intradermal injection.

### Methodology:

An open label, ascending dose evaluation of the safety, tolerability and efficacy of ATX-GD-59 in approximately 12 evaluable subjects with Graves' disease who are HLA-DRB1*15, HLA-DRB1*03 and/or HLA-DRB1*04 positive and not currently treated with anti-thyroid therapy.

"ATX-GD-59" as referred to herein comprises the RNB-5D-K1 and RNB-9B peptides of SEQ ID Nos: 5 and 6.

The first four subjects were recruited sequentially with a minimum of 48 hours between the first dose of each subject. Dosing of the remaining subjects commenced once the first subject had received the second dose and there were no safety concerns identified.

There was an upward titration over five dose levels (injection(s) of 25, 50, 100, 400 and 800 µg) of ATX-GD-59 followed by injection(s) of 800 µg injected on five further occasions. All doses were based on the total peptide.

All doses were administered by intradermal injection at intervals of 14 +/- 3 days.

### Inclusion criteria

1. A diagnosis of Graves' disease as assessed by a physician from clinical and laboratory findings and not receiving anti-thyroid therapy.
2. Quantifiable levels of TSHR antibodies.
3. Raised levels of free T3 and/or free T4 (not exceeding 15 pmol/L and 35 pmol/L respectively) including undetectable levels of thyroid stimulating hormone.
4. HLA-DRB1*15, HLA DRB1*03 and or HLA DRB1*04 positive.
5. Age 18 - 65 years inclusive at the time of informed consent.
6. Subject willing and able to give written informed consent and must be willing to comply with protocol assessments/procedures.
7. Male subjects must be sterile (biologically or surgically) or commit to the use of a reliable method of birth control for the duration of the study until at least 90 days after the last dose of ATX-GD-59.
8. Female subjects of child bearing potential must:
   - neither be pregnant nor breast-feeding, nor attempting to conceive, and
   - use a highly effective method of contraception as defined below, throughout the entire duration of the study and for at least 90 days after the last dose of ATX-GD-59.

### Exclusion criteria

1. Subjects who are pregnant or breastfeeding and/or subjects in the post-partum period.
2. A known history of, or hypersensitivity reactions that in the opinion of the investigator would exclude the subjects' participation in the study.
3. Treatment with any Anti-Thyroid Drugs e.g. carbimazole within the previous 3 months prior to Study Day 1.
4. Previous treatment with radioiodine or (partial or complete) thyroidectomy.
5. Signs of moderate or severe orbitopathy including optic nerve compression requiring steroids and/or a clinical activity score >3.
6. Large and compressive goitres causing localised symptoms such as difficulty swallowing or breathing.
7. Treatment with steroids (administered via the oral and/or parenteral routes) or adrenocorticotropic hormone with the exception of inhaled steroids within the three months prior to Study Day 1.
8. Symptoms and signs of thyroid storm such as confusion, pyrexia with no other cause than hyperthyroidism.
9. Significant cardiac disease and/or atrial fibrillation that would require urgent treatment of thyrotoxicosis.
10. Prior treatment with biological or peptide-based therapeutics including rituximab.
11. Prior use of disease related T cell vaccine or peptide-tolerising agent to treat Graves' disease.
12. Detectable levels of antibodies in plasma specific for any of the peptides within ATX-A history of significant drug allergies.
13. The use of any investigational drug, or participation in any Clinical Trial within three months prior to Study Day 1.
14. Treatment with any cytokine or anti-cytokine therapy within three months prior to Study Day 1.
15. Inadequate liver function, defined by a total bilirubin, aspartate aminotransferase (AST), alanine aminotransferase (ALT) or alkaline phosphatase > 3 times the upper limit of the normal values at Screening visit.
16. Subject with any significant medical illness or psychiatric condition that in the opinion of the Investigator, would preclude participation in the study or impair the ability to give informed consent; any other clinically apparent autoimmune disease.
17. Clinically significant illness, as determined by the investigator, within 4 weeks prior to the first dose (Study Day 1) of ATX-GD-59.
18. Known history of active or chronic infectious disease or any disease which compromises immune function (e.g. HIV+, HTLV-1, Lyme disease, Latent or active TB, Hepatitis).
19. Major surgery in previous four weeks before screening visit.
20. Known osteoporosis or metabolic bone disease.
21. **Withdrawal criteria**
22. Subject experiences a serious or severe adverse event that prevents him/her from continuing.
23. Subject incurs a significant protocol violation that leads to an increased risk to the subject by continuing in the study.
24. Subject requests early discontinuation.
25. Investigator's or Sponsor's request (e.g. if it is considered that the subject's health is compromised by remaining in the study or the subject is not willing or able to comply with the protocol or study procedures).
26. Subject is lost to follow-up.
27. Subject requires treatments prohibited by the exclusion criteria or the protocol.
28. Subject demonstrates a robust and increasing positive response in the anti-drug antibody assay accompanied by clinical signs of an adverse immune response to either peptide in ATX-GD-59. These may be unexplained chills, fever or flu like symptoms following treatment with ATX-GD-59.
29. Subject becomes pregnant.
30. GD-59 at the screening visit.

### Management of thyrotoxicosis during the study

- Symptomatic patients may be treated with propranolol (up to 160mg per day) adjusted to control tachycardia or other symptoms. If alternative beta-adrenergic receptor blockade is required, this must be agreed with the study medical monitor.
- If there is a contraindication to using a beta blocker (e.g., asthma) then rate limiting calcium channel blockers (e.g. Diltiazem MR 120 to 240mg daily) may be used instead. This must also be agreed with the study medical monitor.

### Administration of Anti Thyroid Drug's during the study is indicated in the following instances:

- A subject has trigger levels of free T3 (>20pM) and/or free T4 (>45pM) on 2 consecutive scheduled or unscheduled visits.
- Continued weight loss >5kg from baseline (Study Day 1).
- Persistent tachycardia >120bpm.
- Significant cardiac event that would require urgent treatment of thyrotoxicosis.
- If the investigator or treating physician considers that it is appropriate to administer anti-thyroid drugs at any point during the study.

### Duration of treatment:

The trial consists of a screening visit, a dosing period of 18 weeks, an efficacy assessment after 4 weeks (post final dose) and a follow-up period of 8 weeks.

### Dose and mode of administration:

ATX-GD-59 is a lyophilised equimolar mixture of two peptides reconstituted at the clinic to provide one of two different concentrations, depending on dose, for injection prior to intradermal injection.

**Dose Strengths:** 4mg/ml and 0.5mg/ml total peptide content when reconstituted in water for injection and 0.9% saline respectively.

### Safety monitoring and analysis:

Subjects will be observed in the clinic for at least two hours after administration of the study drug and vital signs will be recorded at 15 minute intervals for the first hour and 30 minute intervals for the second hour. If the subject is well (in the opinion of the Investigator), they will then be allowed to leave the clinic. A contact card with an emergency telephone number will be provided to each subject.

Subjects will be given diaries to take home to record injection site reactions for at least 24 hours after the study drug injection.

The first four subjects will be recruited sequentially with a minimum of 48 hours between the first dose of each subject. Individual subject safety data will be reviewed by the Investigator and the Sponsor or Sponsor's representative after the first dose of ATX-GD-59 and thereafter between the second (week 10) and third (week 12) 800 µg doses. Dosing of the remaining subjects will only commence once the first subject has received the second dose (week 2) 50 µg and there are no safety concerns identified.

For the first 5 subjects, the combined individual subject safety data will be reviewed by the Investigator and the Sponsor or Sponsor's representative after the second 800 µg dose (week10) and prior to the third (week 12) 800µg dose.

The combined safety data for the first 5 subjects will be subject to interim review by a Data Monitoring Committee (DMC) after the last dose of 800 µg (week 18) has been

### Primary Objective

The primary endpoint of this study is a safety assessment to ensure, first of all, that the exposure to the study drug by the intradermal dose route will not harm a population affected by Graves' disease.

Safety endpoints, which will be evaluated on an ongoing basis up to Week 22, are:
- Injection site reactions as reported by the subjects in the diary card.
- Occurrence of treatment related adverse events (Adverse Events), Serious Adverse Events, and laboratory abnormalities compared to baseline.
- Results from the physical examination, and vital signs (reported on an ongoing basis and compared to baseline/screening).
- Premature termination and reasons for premature termination from treatment and/or the study.

### Criteria for Evaluation of the Secondary Objectives:

- TSHR antibody levels.
- Ratio of stimulatory vs inhibitory TSHR antibodies.
- Free T3, free T4 and TSH serum levels.

### Criteria for Evaluation of the Exploratory Objectives:

- TSHR induced PBMC T cell activity.
- T-cell cytokine signature.
- IL-10 pathway and associated genes (mRNA).

### Dosing schedule:

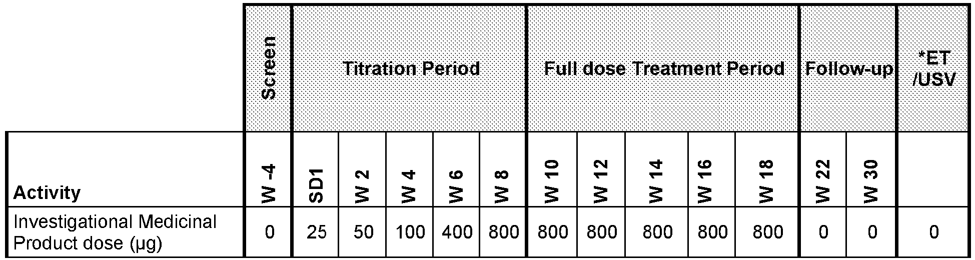

### Results

The safety data were analysed for the first 5 patients by DMC. No safety issues were identified.

In addition, free T3 as well as TSHR Ab level (TRAB) data showed:
- Three of 5 subjects had decreases in T3 and TRAB
- All subjects had decrease in stimulating TRAB levels (TSI)
- There was a strong correlation (0.92) between % change in free T3 and TRAB
- There was a good correlation (0.84) between % change in free T3 and TSI

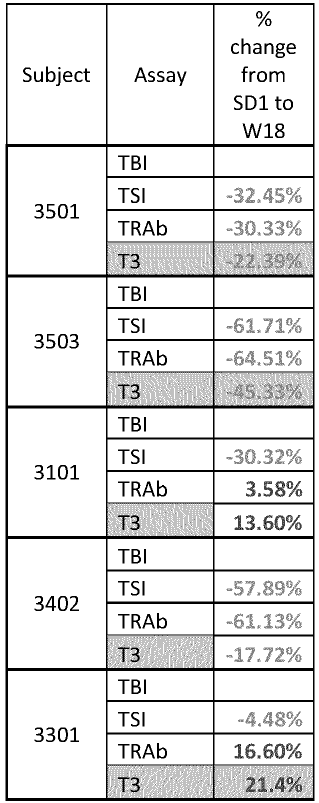

Various modifications and variations of the described methods and uses of the invention will be apparent to those skilled in the art. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology, immunology or related fields are intended to be within the scope of the appended claims, which restrict the scope of the invention.

## Claims

1. A tolerogenic peptide for use in the treatment or prevention of a condition associated with aberrant immune tolerance, wherein
(a) the condition is multiple sclerosis or optic neuritis, and the tolerogenic peptide is selected from myelin basic protein (MBP) peptides MBP 30-44 (SEQ ID NO: 1), MBP 83-99 (SEQ ID NO: 2), MBP 131-145 (SEQ ID NO: 3) and MBP 140-154 (SEQ ID NO: 4); or
(b) the condition is Graves' Disease, and the tolerogenic peptide is selected from thyroid stimulating hormone receptor (TSHR) peptides RNB-5D-K1 (SEQ ID NO: 5) and RNB-9B (SEQ ID NO: 6); and
wherein the tolerogenic peptide is administered via a dose escalation protocol in the following doses:
Day 1: a first dose of 15 to 40µg;
Day 14±7 days: a second dose of 35-65µg;
Day 28±7 days: a third dose of 80-120µg;
Day 42±7 days: a fourth dose of 300-500µg;
Day 56±7 days: a fifth dose of 600-1500µg;
Day 70±7 days: a sixth dose of 600-1500µg;
Day 84±7 days: a seventh dose of 600-1500µg;
Day 98±7 days: an eighth dose of 600-1500µg;
Day 112±7 days: a ninth dose of 600-1500µg; and
Day 126±7 days: a tenth dose of 600-1500µg.

2. The tolerogenic peptide for use according to claim 1
wherein the first dose is 25µg; and/or
wherein the second dose is 50µg; and/or
wherein the third dose is 100µg; and/or
wherein the fourth dose is 400µg.

3. The tolerogenic peptide for use according to claim 1 or claim 2 wherein an eleventh dose of 600-1500µg is administered on day 140±7 days.

4. The tolerogenic peptide for use according to any one of claims 1 to 3 wherein a twelfth dose of 600-1500µg is administered on day 154±7 days.

5. The tolerogenic peptide for use according to claim 4 wherein a thirteenth dose of 600-1500µg is administered on day 168±7 days.

6. The tolerogenic peptide for use according to any one of claims 1 to 5 wherein the fifth, sixth, seventh, eighth, ninth and tenth, and optionally eleventh, twelfth and thirteenth, doses are each 800µg.

7. The tolerogenic peptide for use according to any one of claims 1 to 6 wherein administration of the peptide is intradermal.

8. The tolerogenic peptide for use according to any one of claims 1 to 7 wherein the tolerogenic peptide is administered to a human.

9. The tolerogenic peptide for use according to any one of claims 1 to 8 wherein MBP 30-44 (SEQ ID NO: 1), MBP 83-99 (SEQ ID NO: 2), MBP 131-145 (SEQ ID NO: 3) and MBP 140-154 (SEQ ID NO: 4) are administered.

10. The tolerogenic peptide for use according to any one of claims 1 to 8 wherein RNB-5D-K1 (SEQ ID NO: 5) and RNB-9B (SEQ ID NO: 6) are administered.

## Patentansprüche

1. Tolerogenes Peptid zur Verwendung bei der Behandlung oder Vorbeugung eines Zustands, der mit aberranter Immuntoleranz einhergeht, wobei
(a) der Zustand Multiple Sklerose oder Optikusneuritis ist und das tolerogene Peptid aus den Peptiden des basischen Myelinproteins (MBP) MBP 30-44 (SEQ ID NO: 1), MBP 83-99 (SEQ ID NO: 2), MBP 131-145 (SEQ ID NO: 3) und MBP 140-154 (SEQ ID NO: 4) ausgewählt ist, oder
(b) der Zustand Morbus Basedow ist und das tolerogene Peptid aus den Peptiden des Thyreoidea-stimulierendes-Hormon-Rezeptors (TSHR) RNB-5D-K1 (SEQ ID NO: 5) und RNB-9B (SEQ ID NO: 6) ausgewählt ist, und
wobei das tolerogene Peptid über ein Dosiseskalationsprotokoll in den folgenden Dosen verabreicht wird:
Tag 1: eine erste Dosis von 15 bis 40 ug,
Tag 14±7 Tage: eine zweite Dosis von 35-65 ug,
Tag 28±7 Tage: eine dritte Dosis von 80-120 ug,
Tag 42±7 Tage: eine vierte Dosis von 300-500 ug,
Tag 56±7 Tage: eine fünfte Dosis von 600-1500 ug,
Tag 70±7 Tage: eine sechste Dosis von 600-1500 ug,
Tag 84±7 Tage: eine siebte Dosis von 600-1500 ug,
Tag 98±7 Tage: eine achte Dosis von 600-1500 ug,
Tag 112±7 Tage: eine neunte Dosis von 600-1500 ug und
Tag 126±7 Tage: eine zehnte Dosis von 600-1500 µg.

2. Tolerogenes Peptid zur Verwendung nach Anspruch 1,
wobei die erste Dosis 25 ug beträgt und/oder
wobei die zweite Dosis 50 ug beträgt und/oder
wobei die dritte Dosis 100 ug beträgt und/oder
wobei die vierte Dosis 400 ug beträgt.

3. Tolerogenes Peptid zur Verwendung nach Anspruch 1 oder Anspruch 2, wobei eine elfte Dosis von 600-1500 ug am Tag 140±7 Tage verabreicht wird.

4. Tolerogenes Peptid zur Verwendung nach einem der Ansprüche 1 bis 3, wobei eine zwölfte Dosis von 600-1500 ug am Tag 154±7 Tage verabreicht wird.

5. Tolerogenes Peptid zur Verwendung nach Anspruch 4, wobei eine dreizehnte Dosis von 600-1500 ug am Tag 168±7 Tage verabreicht wird.

6. Tolerogenes Peptid zur Verwendung nach einem der Ansprüche 1 bis 5, wobei die fünfte, sechste, siebte, achte, neunte und zehnte und gegebenenfalls die elfte, zwölfte und dreizehnte Dosis jeweils 800 ug betragen.

7. Tolerogenes Peptid zur Verwendung nach einem der Ansprüche 1 bis 6, wobei die Verabreichung des Peptids intradermal erfolgt.

8. Tolerogenes Peptid zur Verwendung nach einem der Ansprüche 1 bis 7, wobei das tolerogene Peptid an einen Menschen verabreicht wird.

9. Tolerogenes Peptid zur Verwendung nach einem der Ansprüche 1 bis 8, wobei MBP 30-44 (SEQ ID NO: 1), MBP 83-99 (SEQ ID NO: 2), MBP 131-145 (SEQ ID NO: 3) und MBP 140-154 (SEQ ID NO: 4) verabreicht werden.

10. Tolerogenes Peptid zur Verwendung nach einem der Ansprüche 1 bis 8, wobei RNB-5D-K1 (SEQ ID NO: 5) und RNB-9B (SEQ ID NO: 6) verabreicht werden.

## Revendications

1. Peptide tolérogénique pour une utilisation dans le traitement ou la prévention d'une pathologie associée à une tolérance immunitaire aberrante,
(a) la pathologie étant une sclérose en plaques ou une névrite optique, et le peptide tolérogénique étant choisi parmi des peptides de la protéine basique de la myéline (MBP) MBP 30-44 (SEQ ID NO: 1), MBP 83-99 (SEQ ID NO: 2), MBP 131-145 (SEQ ID NO: 3) et MBP 140-154 (SEQ ID NO: 4) ; ou
(b) la pathologie étant la maladie de Graves, et le peptide tolérogénique étant choisi parmi des peptides du récepteur de stimulation de la thyroïde (TSHH) RNB-5D-K1 (SEQ ID NO: 5) et RNB-9B (SEQ ID NO: 6) ; et
le peptide tolérogénique étant administré via un protocole d'augmentation de dose en les doses suivantes
Jour 1 : première dose de 15 à 40 ug ;
Jour 14 ± 7 jours : une deuxième dose de 35 à 65 ug ;
Jour 28 ± 7 jours : une troisième dose de 80 à 120 ug ;
Jour 42 ± 7 jours : une quatrième dose de 300 à 500 µg ;
Jour 56 ± 7 jours : une cinquième dose de 600 à 1 500 µg ;
Jour 70 ± 7 jours : une sixième dose de 600 à 1 500 µg ;
Jour 84 ± 7 jours : une septième dose de 600 à 1 500 µg ;
Jour 98 ± 7 jours : une huitième dose de 600 à 1 500 µg ;
Jour 112 ± 7 jours : une neuvième dose de 600 à 1 500 µg ; et
Jour 126 ± 7 jours : une dixième dose de 600 à 1 500 ug.

2. Peptide tolérogénique pour une utilisation selon la revendication 1
la première dose étant de 25 µg ; et/ou
la deuxième dose étant de 50 µg ; et/ou
la troisième dose étant de 100 µg ; et/ou
la quatrième dose étant de 400 ug.

3. Peptide tolérogénique pour une utilisation selon la revendication 1 ou la revendication 2, une onzième dose de 600 à 1 500 µg étant administrée au jour 140 ± 7 jours.

4. Peptide tolérogénique pour une utilisation selon l'une quelconque des revendications 1 à 3, une douzième dose de 600 à 1 500 µg étant administrée au jour 154 ± 7 jours.

5. Peptide tolérogénique pour une utilisation selon la revendication 4, une treizième dose de 600 à 1 500 µg étant administrée au jour 168 ± 7 jours.

6. Peptide tolérogénique pour une utilisation selon l'une quelconque des revendications 1 à 5, les cinquième, sixième, septième, huitième, neuvième et dixième, et éventuellement onzième, douzième et treizième, doses étant chacune de 800 µg.

7. Peptide tolérogénique pour une utilisation selon l'une quelconque des revendications 1 à 6, une administration du peptide étant intradermique.

8. Peptide tolérogénique pour une utilisation selon l'une quelconque des revendications 1 à 7, le peptide tolérogénique étant administré à un humain.

9. Peptide tolérogénique pour une utilisation selon l'une quelconque des revendications 1 à 8, MBP 30-44 (SEQ ID NO: 1), MBP 83-99 (SEQ ID NO: 2), MBP 131-145 (SEQ ID NO: 3) et MBP 140-154 (SEQ ID NO: 4) étant administrés.

10. Peptide tolérogénique pour une utilisation selon l'une quelconque des revendications 1 à 8, RNB-5D-K1 (SEQ ID NO: 5) et RNB-9B (SEQ ID NO: 6) étant administrés.
